# EUROPÄISCHE PATENTSCHRIFT

(11) **EP 3 972 478 B1**
(45) Veröffentlichungstag und Bekanntmachung des Hinweises auf die Patenterteilung: **18.01.2023**
(21) Anmeldenummer: 20757600.0
(22) Anmeldetag: 21.08.2020
(51) Int. Cl.: A61B 3/09, A61B 3/028

(54) **GEMEINSAME BESTIMMUNG VON AKKOMMODATION UND VERGENZ**
JOINT DETERMINATION OF ACCOMMODATION AND VERGENCE
DÉTERMINATION COMMUNE DU POUVOIR D'ACCOMMODATION ET DE LA VERGENCE

(30) Priorität: 23.08.2019 EP 19193432
(43) Veröffentlichungstag der Anmeldung: 30.03.2022
(60) Teilanmeldung: 21216841.3
(73) Patentinhaber: Carl Zeiss Vision International GmbH, 73430 Aalen (DE)
(72) Erfinder: LEUBE, Alexander, 73434 Aalen (DE); OHLENDORF, Arne, 72074 Tübingen (DE); WAHL, Siegfried, 73072 Donzdorf (DE)
(74) Vertreter: Altmann Stößel Dick Patentanwälte PartG mbB
(86) Internationale Anmeldenummer: PCT/EP2020/073494
(87) Internationale Veröffentlichungsnummer: WO 2021/037718

(56) Entgegenhaltungen:
- WO-A1-2009/007136
- DE-A1-102012 022 662
- US-A1- 2013 176 534
- JANE GWIAZDA ET AL: "Response AC/A ratios are elevated in myopic children", OPHTHALMIC AND PHYSIOLOGICAL OPTICS., Bd. 19, Nr. 2, 1. März 1999 (1999-03-01), Seiten 173-179, XP055671285, GB ISSN: 0275-5408, DOI: 10.1046/j.1475-1313.1999.00437.x

## Beschreibung

### Gebiet der Erfindung

Die vorliegende Erfindung betrifft ein Verfahren und eine Vorrichtung zur gemeinsamen Bestimmung von Akkommodation und Vergenz mindestens eines Auges eines Nutzers, eine Vorrichtung zur Datenverarbeitung sowie ein Computerprogramm, einen computerlesbaren Datenträger, Datenträgersignale und ein computerlesbares Medium. Die vorliegende Erfindung kann somit insbesondere zur Myopie-Kontrolle eingesetzt werden; weitere Einsatzbereiche sind jedoch denkbar.

### Stand der Technik

Aus dem Stand der Technik sind Verfahren und Vorrichtungen zur Bestimmung von Akkommodation und Vergenz eines Auges oder beider Augen eines Nutzers, insbesondere zur Myopie-Kontrolle, bekannt. Die Myopie-Kontrolle wird jedoch meist standardisiert durchgeführt, ohne dass hierbei ein Einfluss individueller physiologischer Parameter berücksichtigt wird. In klinischen Anwendungen werden z.B. für Gleitsicht-Brillengläser, die eine Naheinstellung des Auges unterstützen und auf diese Weise ein Voranschreiten einer Kurzsichtigkeit (Myopie) verlangsamen sollen, standardmäßige Additionswerte und/oder Insetwerte verwendet, individuell erfasste Parameter in Bezug auf Akkommodation und/oder Vergenz jedoch außer Acht gelassen. Information über die Genauigkeit der Akkommodation und einer hieraus sich ergebenden Naheinstellung der Augen werden im bisherigen klinischen Alltag durch subjektive und/oder objektive Methoden gewonnen. Derartige Methoden, wie z.B. Messung einer Nahfehlstellung der Augen durch Vorsatzprismen, erfordern allerdings trainiertes Personal und eine subjektive Einschätzung insbesondere von Unschärfe oder Einstellbewegung der Augen. Aus dem Stand der Technik bekannte Vorrichtungen und Verfahren bestimmen die Akkommodation und die Vergenz der Augen eines Nutzers jeweils auf getrennte Weise, wobei zusätzlich angenommen wird, dass der Aufwand jeden Auges zur Akkommodation genau einem Stimulus-Wert entspricht.

Jedoch können die Akkommodation und die Vergenz der Augen eines Nutzers nicht völlig unabhängig voneinander betrachtet werden. In Messungen zur Genauigkeit der Akkommodation des Auges konnte nachgewiesen werden, dass eine Verringerung eines Akkommodationsfehlers mittels Gleitsicht-Brillengläsern signifikant von einer gewählten Addition abhängig ist. Gwiazda J., Thorn F., Bauer J. und Held R., Myopic Children Show Insufficient Accommodative Response to Blur, Investigative Ophthalmology & Visual Science 34(3), 1993, S. 690-94 konnten zeigen, dass eine Ungenauigkeit der Akkommodation kurzsichtiger Probanden diejenige von nicht-kurzsichtigen Probanden übersteigt. Weiterhin ist nach Gwiazda J., Thorn F., und Held R., Accommodation, Accommodative Convergence, and Response AC/A Ratios Before and at the Onset of Myopia in Children, Optometry and Vision Science 82(4), 2005, S. 273-78, der Betrag der Vergenz, welcher für eine bestimmte Akkommodationsentfernung eingestellt wird (abgekürzt "AC/A" von engl. *accommodative convergence*/*accommodation*), bei Kurzsichtigen höher als der tatsachlich benötigte Betrag, so dass der resultierende Fehler größer als bei nicht-kurzsichtigen Probanden ist.

Win-Hall D.M., und Glasser, A., Objective accommodation measurements inprepres-byopic eyes using an autorefractor and an aberrometer, J. Cataract. Refract. Surg. 34(5), 2008, S. 774-84, führten eine Studie zur Ermittlung der Wiederholbarkeit der Bestimmung der Akkommodation mittels eines Aberrometers und in einem Autorefraktor in jungen, phakischen, präpresbyopischen Personen durch. Die Akkommodation wurde mittels Zeichen in verschiedenen Abständen angeregt. Die Studie ergab, dass sich die auf beide Weise ermittelte Akkommodation nicht signifikant voneinander unterscheidet und sich daher zur objektiven Bestimmung der Akkommodation in einer phakischen, präpresbyopischen Population mit geringen akkommodativen Amplituden eignet.

US 5,684,561 A offenbart einen Autorefraktor, der zwei Lichtquellen und eine zugehörige Optik zur Projektion eines Bildes auf den Augenhintergrund umfasst, wobei ein einzelner Detektor ein Signal erzeugt, das jeder Lichtquelle entspricht. In einer alternativen Ausgestaltung werden eine einzelne Lichtquelle und zwei Detektoren eingesetzt. Das von dem Augenhintergrund reflektierte Licht wird nachgewiesen und Unterschiede in den beiden Signalen werden dazu verwendet, Abweichungen von einer Null-Dioptrien-Sphäre zu bestimmen. Segmentierte oder CCD-Detektoren werden dazu verwendet, Zylinder, Achse, Länge und Blickrichtung zu bestimmen und hieraus ein Netzhautbild zu ermitteln und zu analysieren.

US 7,290,879 B2 offenbart eine kombinierte Vorrichtung zur Bestimmung der Refraktion- und Akkommodationsfunktion des Auges. Die Vorrichtung zur Bestimmung der Refraktion weist eine Schalteinrichtung auf, mittels welcher zwischen zwei unterschiedlichen Messarten, umfassend eine normale Messung der Refraktion, welche die sphärische Refraktion, die zylindrische Refraktion und die astigmatische Achse erfasst, und eine Messung der Akkommodationsfunktion, welche eine Änderung in der Refraktion des Auges für Hochfrequenz-Komponenten erfasst, gewählt werden kann.

US 2012/0287398 A1 offenbart eine binokulare Sichtanalyse-Vorrichtung zur Bestimmung einer Verschreibung von Augenhilfsmitteln für die Augen eines Nutzers. Die Vorrichtung umfasst ein optisches System, welches dazu eingerichtet ist, virtuelle Bilder für jeweils ein für eines der beiden Augen sichtbares Ziel darzustellen. Mindestens ein Strahlteiler, welcher vor jedem der beiden Augen angeordnet ist, führt die virtuellen Bilder zu den entsprechenden Augen. Die Vorrichtung umfasst ferner Einrichtungen zur Sphärenkorrektur sowie zur zylindrischen Korrektur, welche jeweils einem der beiden Augen zugeordnet sind und aus welchen die jeweilige Refraktion des Auges ermittelbar ist. Eine optionale Einrichtung zur Ermittlung von Augenfolgebewegungen kann zur Aufnahme der Augenpositionen und hieraus zur Anpassung der Position des optischen Systems eingesetzt werden.

Weiterhin sind Verfahren und Vorrichtung zur Bestimmung von Augenbewegungen eines Nutzers bekannt.

US 6,402,320 B1 offenbart ein automatisches Verfahren zur Bestimmung einer visuellen Sehschärfe insbesondere für Kleinkinder mittels einer elektronischen visuellen Anzeigeeinrichtung, das folgende Schritte umfasst: (a) Bereitstellen eines Fixationsziels auf der Anzeigeeinrichtung als Stimulus für den Nutzer; dann (b) Bereitstellen eines Testbildes auf der Anzeigeeinrichtung, wobei das Testbild mindestens zwei separate Felder umfasst, wobei eines der Felder ein erstes Testmuster und ein anderes der Felder ein Kontrollmuster aufweist, wobei das Testmuster als Stimulus für den Nutzer eingerichtet ist, wenn das Testmuster für den Nutzer erkennbar ist; dann (c) Nachweisen, ob eine Augenbewegung zu dem Testmuster auftritt, wobei ein Auftreten einer Augenbewegung zu dem Testmuster die Unterscheidbarkeit des ersten Testmusters durch den Nutzer bestätigt; dann (d) Wiederholen der Schritte (b) und (c) mit einem weiteren Testmuster, wobei das weitere Testmuster schwerer unterscheidbar ist als das erste Testmuster; und (e) Bestimmen der visuellen Sehschärfe des Nutzers aus dem Auftreten oder der Abwesenheit der Augenbewegung zu dem ersten Testmuster und zu wenigstens einem weiteren Testmuster.

US 2015/070273 A1 offenbart Verfahren und Vorrichtungen zum optischen Nachweis und Verfolgen einer Augenbewegung. Ein Verfahren zur Verfolgung der Augenbewegung umfasst Emittieren von Licht auf das Auge des Nutzers unter Verwendung von mehreren, im Wesentlichen gleichweit von einem Photodetektor-Modul der Vorrichtung entfernten Lichtquellen, Empfangen Modul einer wenigstens teilweisen Rückstrahlung des von jeder der mehreren Lichtquellen emittierten und von dem Auge zurückgestrahlten Lichts in dem Photodetektor, und Bestimmen eines Positionsparameters des Auges basierend auf verschiedenen Werten der wenigstens teilweisen Rückstrahlung des Lichts in Bezug auf die mehreren Lichtquellen.

DE 10 2012 022 662 A1 offenbart eine Vorrichtung sowie ein Verfahren zur Überprüfung der menschlichen Sehfähigkeit, umfassend ein Bilderzeugungsmodul zur Erzeugung beliebiger Testbilder, ein Abbildungsmodul, welches dazu dient, das von dem Bilderzeugungsmodul bereitgestellte Testbild als Stimulus auf der Netzhaut des Auges abzubilden, wobei das Abbildungsmodul mindestens ein optisches Bauelement mit variabler Brennweite enthält, sodass das Testbild des Bilderzeugungsmoduls für das Auge aus virtuell nachgebildeten und variablen Entfernungen wahrnehmbar ist, eine Akkommodationsmesseinrichtung zur Messung der Akkommodation des Auges, eine Blickrichtungsmesseinrichtung zur Messung der Blickrichtung des Auges, ein Steuerungs- und Auswertemodul, welches die von den einzelnen Modulen stammenden Informationen und/oder Messwerte erfasst und/oder weiterverarbeitet und/oder den Betriebsablauf steuert. Die Vorrichtung zeichnet sich dadurch aus, dass mittels des jeweiligen Testbilds eine Stimulation der Akkommodation des Auges und/oder mittels der Blickrichtung durchführbar ist, die Messung der Akkommodation des Auges sowie Blickrichtung des Auges gleichzeitig oder alternierend durchführbar ist und die Messwerte der Akkommodation des Auges sowie der Blickrichtung des Auges einem Steuerungs- und Auswertemodul zuführbar sind. In einer besonderen Ausgestaltung der Vorrichtung kann eine simultane Überprüfung beider Augen unter Erzeugung von virtuellen binokulären Bildern vorgenommen werden, die sich z. B. in sehphysiologischer Hinsicht nicht von der Seh-Wirklichkeit unterscheiden lassen.

WO 2009/007136 A1 offenbart ein Verfahren zum Überprüfen und/oder Bestimmen von Benutzerdaten eines Brillenglasbenutzers mit den Schritten: Bereitstellen subjektiver Daten eines Brillenglasbenutzers, wobei die subjektiven Daten zumindest subjektive Refraktionsdaten umfassen; Bereitstellen objektiver Refraktionsdaten des Brillenglasbenutzers; Vergleichen zumindest einer Teilmenge der subjektiven Refraktionsdaten mit zumindest einer Teilmenge der objektiven Refraktionsdaten und Bestimmen eines Vergleichsergebnisses; Anpassen zumindest der Teilmenge der subjektiven Refraktionsdaten an die objektiven Refraktionsdaten aufgrund des Vergleichsergebnisses unter der Voraussetzung, dass das Vergleichsergebnis zumindest eine vorbestimmte Vergleichsbedingung erfüllt, ansonsten Beibehalten zumindest der Teilmenge der subjektiven Refraktionsdaten und/oder Bereitstellen einer Nachricht, welche das Vergleichsergebnis enthält.

Europäische Patentanmeldung Az. 19 170 561.5, angemeldet am 23.04.2019, offenbart ein Verfahren zur Bestimmung eines Refraktionsfehlers eines Auges eines Nutzers. Hierzu erfolgt ein Darstellen eines Zeichens auf einem Bildschirm, wobei ein Parameter des auf dem Bildschirm dargestellten Zeichens verändert wird, ein Erfassen einer Augenbewegungsmetrik des Auges des Nutzers in Abhängigkeit von dem auf dem Bildschirm dargestellten Zeichen, ein Feststellen eines Zeitpunkts, zu dem sich aus der Augenbewegungsmetrik des Auges des Nutzers eine Erkennungsschwelle des Nutzers für das auf dem Bildschirm dargestellte Zeichen ergibt, und ein Bestimmen eines Wertes für den Refraktionsfehler des Auges des Nutzers aus dem zu dem Zeitpunkt festgelegten Parameter.

Europäische Patentanmeldung Az. 19 182 861.5, angemeldet am 27.06.2019, offenbart ein Verfahren und eine Vorrichtung zur Bestimmung einer Kontrastempfindlichkeitsschwelle von Augen eines Nutzers. Hierzu werden Augenbewegungen, die durch einen Stimulus, der zur Anregung eines optokinetischen Nystagmus eingerichtet ist, aufgenommen und ausgewertet.

### Aufgabe der Erfindung

Insbesondere ausgehend von der Offenbarung der US 2012/0287398 A1 besteht die Aufgabe der vorliegenden Erfindung darin, ein Verfahren und eine Vorrichtung zur gemeinsamen Bestimmung von Akkommodation und Vergenz mindestens eines Auges eines Nutzers, eine Vorrichtung zur Datenverarbeitung sowie ein Computerprogramm, einen computerlesbaren Datenträger, Datenträgersignale und ein computerlesbares Medium bereitzustellen, welche die aufgeführten Nachteile und Einschränkungen des Standes der Technik zumindest teilweise überwinden.

Insbesondere sollen die Vorrichtung, das Verfahren und das Computerprogramm die gemeinsame Bestimmung der Akkommodation und der Vergenz mindestens eines Auges des Nutzers, bevorzugt der beiden Augen des Nutzers, sowie die Myopie-Kontrolle mindestens eines Auges des Nutzers, bevorzugt der beiden Augen des Nutzers, ermöglichen, ohne dass auf eine subjektive Einschätzung von entsprechend trainiertem Personal zurückgegriffen werden muss.

Weiterhin soll die gemeinsame Bestimmung der Akkommodation und der Vergenz mindestens eines Auges des Nutzers, bevorzugt der Augen des Nutzers, als Basis für eine effektive Myopie-Kontrolle dienen können, und zwar sowohl für eine Erstversorgung des Nutzers als auch zur Verlaufskontrolle insbesondere im Hinblick auf eine potentielle Myopie-Progression bei dem Nutzer.

### Offenbarung der Erfindung

Diese Aufgabe wird gelöst durch eine Erfindung mit den Merkmalen der unabhängigen Patentansprüche. Bevorzugte Ausgestaltungen, welche einzeln oder in Kombination realisierbar sind, sind in den abhängigen Ansprüchen dargestellt.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben dem durch diese Begriffe eingeführten Merkmal, keine weiteren Merkmale vorhanden sind oder auf Situationen, in welchen ein oder mehrere weitere Merkmale vorhanden sind.

In einem ersten Aspekt betrifft die vorliegende Erfindung ein Verfahren zur gemeinsamen Bestimmung von Akkommodation und Vergenz mindestens eines Auges eines Nutzers. Das Verfahren umfasst die folgenden Schritte a) bis d), vorzugsweise in der angegebenen Reihenfolge. Auch eine andere Reihenfolge ist grundsätzlich möglich. Insbesondere ist auch eine ganz oder teilweise zeitgleiche Ausführung der Schritte möglich. Weiterhin können einzelne, mehrere oder alle Schritte des Verfahrens wiederholt, insbesondere mehr als einmal, ausgeführt werden. Das Verfahren kann, zusätzlich zu den genannten Schritten auch weitere Verfahrensschritte umfassen.

Das Verfahren zur gemeinsamen Bestimmung von Akkommodation und Vergenz mindestens eines Auges eines Nutzers umfasst die Schritte:
a) Darstellen mindestens eines Zeichens in mindestens einer ersten Entfernung vor mindestens einem Auge eines Nutzers zur Stimulation der Akkommodation des mindestens einen Auges;
b) Erfassen mindestens einer Augenbewegung des mindestens einen Auges;
c) Ermitteln einer Refraktion des mindestens einen Auges bei der Akkommodation des mindestens einen Auges in die mindestens eine erste Entfernung; und
d) gemeinsames Bestimmen der Akkommodation und der Vergenz des mindestens einen Auges durch
   o Ermitteln einer Änderung der Refraktion des mindestens einen Auges bei der Akkommodation des mindestens einen Auges in die mindestens eine erste Entfernung gegenüber der Akkommodation des mindestens einen Auges in mindestens eine zweite Entfernung; und
   o Ermitteln der Vergenz des mindestens einen Auges aus der mindestens einen Augenbewegung des mindestens einen Auges bei der Akkommodation des mindestens einen Auges in die mindestens eine erste Entfernung.

Bevorzugt werden mittels vorstehend aufgeführtem Verfahren die Akkommodation und die Vergenz der Augen des Nutzers, besonders bevorzugt gleichzeitig, bestimmt.

Bei einer bevorzugten Ausführungsform werden die einzelnen Schritte des vorstehend aufgeführten Verfahrens zur Bestimmung der Akkomodation und der Vergenz mindestens eines Auges eines Nutzers mit Hilfe wenigstens eines mobilen Endgeräts ausgeführt. Unter wenigstens einem mobilen Endgerät ist bevorzugt eine Vorrichtung zu verstehen, welche zumindest einen programmierbaren Prozessor sowie wenigstens eine Kamera und wenigstens einen Beschleunigungssensor umfasst, und welche bevorzugt dazu ausgelegt ist, getragen zu werden, d.h. hinsichtlich Dimensionen und Gewicht derart ausgestaltet ist, dass sie von einer Person mitführbar ist. In dem wenigstens einen mobilen Endgerät können weitere Komponenten vorhanden sein, wie zum Beispiel wenigstens ein Bildschirm, wenigstens eine Lichtquelle für beispielsweise sichtbares Licht aus einem Wellenlängenbereich von 380 nm bis 780 nm und/oder infrarotes Licht aus einem Wellenlängenbereich von 780 nm bis 1 mm und/oder wenigstens einen Lichtempfänger mit einer Sensibilität für beispielsweise sichtbares Licht aus einem Wellenlängenbereich von 380 nm bis 780 nm und/oder infrarotes Licht aus einem Wellenlängenbereich von >780 nm bis 1 mm. Typische Beispiele für derartige mobile Endgeräte sind Smartphones oder Tablet-PCs, die wenigstens einen Bildschirm, beispielsweise einen Sensorbildschirm (Touchscreen), wenigstens eine Kamera, wenigstens einen Beschleunigungssensor, wenigstens eine Lichtquelle, wenigstens einen Lichtempfänger und weitere Komponenten, wie drahtlosten Schnittstellen für Mobilfunk oder WLAN (Wireless LAN) aufweisen können. Das Darstellen mindestens eines Zeichens in mindestens einer ersten Entfernung vor mindestens einem Auge eines Nutzers zur Stimulation der Akkommodation des mindestens einen Auges gemäß Schritt a) des erfindungsgemäßen Verfahrens kann beispielsweise mittels des wenigstens einen Bildschirms des wenigstens einen mobilen Endgeräts erfolgen. Das Erfassen einer Augenbewegung des mindestens einen Auges gemäß Schritt b) des erfindungsgemäßen Verfahrens kann beispielsweise mittels der wenigstens einen Kamera; oder mittels der wenigstens einen Lichtquelle und mittels der wenigstens einen Kamera oder dem wenigstens einen Lichtempfänger, jeweils des wenigstens einen mobilen Endgeräts, erfolgen. Das Ermitteln einer Refraktion des mindestens einen Auges bei der Akkommodation des mindestens einen Auges in die mindestens eine erste Entfernung gemäß Schritt c) des erfindungsgemäßen Verfahrens kann beispielsweise mittels der wenigstens einen Kamera; oder mittels der wenigstens einen Lichtquelle und mittels der wenigstens einen Kamera oder dem wenigstens einen Lichtempfänger, jeweils des wenigstens einen mobilen Endgeräts, erfolgen. Das gemeinsame Bestimmen der Akkommodation und der Vergenz des mindestens einen Auges durch
- Ermitteln einer Änderung der Refraktion des mindestens einen Auges bei der Akkommodation des mindestens einen Auges in die mindestens eine erste Entfernung gegenüber der Akkommodation des mindestens einen Auges in eine zweite Entfernung; und
- Ermitteln der Vergenz des mindestens einen Auges aus der mindestens einen Augenbewegung des mindestens einen Auges bei der Akkommodation des mindestens einen Auges in die mindestens eine erste Entfernung gemäß Schritt d) des erfindungsgemäßen Verfahrens kann beispielsweise mittels der wenigstens einen Kamera; oder mittels der wenigstens einen Lichtquelle und mittels der wenigstens einen Kamera oder dem wenigstens einen Lichtempfänger, jeweils des wenigstens einen mobilen Endgeräts, erfolgen.

Der Begriff "Akkommodation" betrifft eine Anpassung der Refraktion mindestens eines Auges eines Nutzers bei einer Abbildung eines Objekts, das sich in einer grundsätzlich beliebigen Entfernung vor mindestens einem Auge des Nutzers zwischen dem Nahpunkt und dem Fernpunkt befindet, auf die Netzhautebene des mindestens einen Auges. Hierbei betrifft der "Fernpunkt" einen Endpunkt einer Refraktionsrichtung des mindestens einen Auges des Nutzers im akkommodationslosen Zustand. Im Rahmen der vorliegenden Erfindung umfasst der Begriff "Akkommodation" auch den akkommodationslosen Zustand, welcher mittels mindestens eines Zeichens, das sich im Fernpunkt befindet, stimuliert wird. Im Gegensatz hierzu bezeichnet der "Nahpunkt" einen Punkt, der die kleinste Entfernung vor dem mindestens einen Auge des Nutzers angibt, bei der das Objekt noch scharf auf die Netzhautebene des mindestens einen Auges abgebildet werden kann, wobei der Nahpunkt eine insbesondere vom Alter des Nutzers abhängige individuelle Größe darstellt. Als Bezugspunkt für die Messung der Entfernung kann eine festgelegte Stelle des mindestens einen Auges, insbesondere auf der Hornhaut, beispielsweise ein Ort eines beobachtbaren Hornhautreflexes, dienen.

Der Begriff "Refraktion" bezeichnet hierbei eine Lichtbrechung im mindestens einen Auge des Nutzers, die ein durch die Pupille in das Innere des mindestens einen Auges einfallender Lichtstrahl erfährt. Die Defokussierung des mindestens einen Auges des Nutzers kann zu einer Fehlsichtigkeit (Ametropie) des Nutzers führen, insbesondere zu einer Kurzsichtigkeit (Myopie) oder zu einer Weitsichtigkeit (Hyperopie) des Nutzers. Zur aus dem Stand der Technik bekannten subjektiven Bestimmung der Refraktion werden üblicherweise Sehzeichen, bevorzugt in Form von Zahlen, Buchstaben oder Symbolen, auf einer Tafel oder einem Bildschirm in einer festgelegten Größe für eine gegebene Entfernung bereitgestellt, die der Nutzer betrachtet. Durch ein Vorhalten einer Anzahl von optischen Linsen mit bekannten Eigenschaften sowie durch eine Führung des Nutzers in einem definierten Frageprozess kann subjektiv bestimmt werden, welche Defokussierung das mindestens eine Auge des Nutzers aufweist und welche refraktive Ausgestaltung des Brillenglases zu einem weitgehenden Ausgleich der Defokussierung des mindestens einen ametropen Auges des Nutzers und damit zu einer möglichst optimalen Bildqualität für den Nutzer führt. Der Begriff der "Brille" bezeichnet hierbei ein beliebiges Element, welches zwei einzelne Brillengläser und eine Brillenfassung umfasst, wobei das Brillenglas zur Einbringung in eine Brillenfassung vorgesehen ist, welche von einem Nutzer der Brille ausgewählt wird. Anstelle des hier verwendeten Begriffs des "Nutzers" kann gleichbedeutend auch einer der Begriffe "Subjekt", "Brillenträger", "Benutzer" oder "Proband" verwendet werden.

Der Begriff "Vergenz" bezeichnet gegensinnige Augenbewegungen der beiden Augen eines Augenpaars des Nutzers, wobei jedes der beiden Augen eine Augenrotation um zueinander parallele Achsen in eine jeweils entgegengesetzte Rotationsrichtung ausführt. Jede dieser zueinander parallelen Achsen stellt hierbei jeweils eine Verlängerung ins Unendliche der Verbindungslinie zwischen dem Zentrum der Pupille eines Auges und dessen Augendrehpunkt dar. Der Augendrehpunkt ist hierbei das geometrische Rotationszentrum des Auges. Unter den Begriff "Vergenz" fällt sowohl die gegensinnige Augenbewegung der beiden Augen eines Augenpaars des Nutzers zur Mittellinie als auch die gegensinnige Augenbewegung der beiden Augen eines Augenpaars des Nutzers divergent weg von der Mittellinie. Die Mittellinie bezeichnet die senkrechte Projektion ins Unendliche in halber Pupillendistanz senkrecht zur Strecke der Pupillendistanz. Das Zentrum der Pupille ist der geometrische Mittelpunkt der Pupille.

Der Begriff "Vergenz" bezeichnet weiterhin die Augenbewegung mindestens eines Auges des Nutzers von der Achse, welche die Verlängerung der Verbindungslinie ins Unendliche zwischen dem Zentrum der Pupille eines Auges und dessen Augendrehpunkt darstellt, sowohl zur Mittellinie als auch divergent weg von dieser. Weiterhin sind vom Begriff "Vergenz" auch Augenbewegungen der beiden Augen eines Augenpaars des Nutzers umfasst, wobei jedes der beiden Augen unabhängig voneinander eine Augenrotation um seine jeweilige Achse, welche die Verlängerung ins Unendliche der Verbindungslinie zwischen dem Zentrum der Pupille eines Auges und dessen Augendrehpunkt darstellt, sowohl zur Mittellinie als auch divergent weg von dieser ausführt. In allen vorstehend genannten Ausführungen zur Vergenz und Divergenz beider Augen kann der Betrag der Augenrotation der beiden Augen unterschiedlich ausgeprägt sein. Diese unterschiedliche Ausprägung kann sowohl dann vorliegen, wenn die Augenrotation beider Augen vergent oder divergent ist als auch dann, wenn die Augenrotation eines der beiden Augen vergent und die Augenrotation des anderen Auges divergent ist.

Wie eingangs bereits erwähnt, können die Akkommodation und die Vergenz des mindestens einen Auges des Nutzers, bevorzugt des Augenpaars des Nutzers, nicht völlig unabhängig voneinander betrachtet werden. Für einen definierten Akkommodationsaufwand ist dies jeweils mit einem entsprechenden Vergenzaufwand verbunden. Vielmehr ist eine Verringerung eines Akkommodationsfehlers mittels Gleitsicht-Brillengläsern signifikant von einer gewählten Addition abhängig. Ebenso übersteigt eine Ungenauigkeit der Akkommodation von kurzsichtigen Probanden diejenige von nicht-kurzsichtigen Probanden. Weiterhin ist der Betrag der Vergenz, welcher für eine bestimmte Akkommodationsentfernung eingestellt wird (auch abgekürzt "AC/A" von engl. *accommodative convergence*/*accommodation*), bei Kurzsichtigen höher als der tatsachlich benötigte Betrag, so dass der resultierende Fehler größer als bei nicht-kurzsichtigen Probanden ist. Darüber hinaus umfasst der Begriff "Vergenz" im Zusammenhang mit der vorliegenden Erfindung auch eine so genannte "Divergenz", welche auftritt, wenn die Entfernung vom Nahpunkt zum Fernpunkt geändert wird, bei welcher die Augen ebenfalls eine gegensinnige Rotationsbewegung um zueinander parallele Achsen divergent weg von der Mittellinie oder bei welcher die Augen eine voneinander unabhängige Augenrotation um die jeweilige Achse des Auges divergent weg von der Mittellinie ausführen. Weiterhin umfasst der Begriff "Vergenz" auch eine "Divergenz", welche auftritt, wenn die Entfernung vom Nahpunkt zum Fernpunkt geändert wird und mindestens ein Auge des Nutzers eine Augenbewegung um die Achse, welche die Verlängerung der Verbindungslinie ins Unendliche zwischen dem Zentrum der Pupille eines Auges und dessen Augendrehpunkt darstellt, divergent von der Mittellinie weg ausführt. Mit Achse ist auch hier, wie vorstehend definiert, jeweils die Verlängerung ins Unendliche der Verbindungslinie zwischen dem Zentrum der Pupille eines Auges und dessen Augendrehpunkt gemeint. Die Mittellinie ist auch hier, wie bereits ebenfalls vorstehend definiert, jeweils die senkrechte Projektion ins Unendliche in halber Pupillendistanz senkrecht zur Strecke der Pupillendistanz.

Insbesondere um den Quotienten AC/A, der durch den Betrag der Vergenz definiert ist, den das mindestens eine Auge selbsttätig für eine bestimmte Akkommodationsentfernung einstellt, möglichst exakt zu bestimmen, erfolgt erfindungsgemäß die Bestimmung der beiden Größen Akkommodation und Vergenz eines oder beider Augen des Nutzers gemeinsam. Die Begriffe "Bestimmen", "Bestimmung", "Ermitteln" und "Ermittlung" bezeichnen hierbei eine Berechnung eines Wertes, der aus mindestens einer messtechnisch erfassbaren Messgröße, die mit dem Wert in Verbindung steht, insbesondere unter Verwendung einer Auswerteeinheit, abgeleitet werden kann. Der Begriff der "Erfassung" bezieht sich hierbei auf eine Aufnahme der mindestens einen, messtechnisch erfassbaren Messgröße, aus welcher, insbesondere unter Verwendung der Auswerteeinheit, der gewünschte Wert abgeleitet werden kann. Der Begriff "gemeinsam" bezeichnet hierbei die Bestimmung der beiden Messgrößen in einem engen zeitlichen Zusammenhang, bevorzugt unter Verwendung der von derselben Vorrichtung erfassten Messgrößen, besonders bevorzugt in einem zeitlichen Zusammenhang, insbesondere gleichzeitig oder unmittelbar aufeinanderfolgend. Auf diese Weise kann erfindungsgemäß der oben erwähnte enge Zusammenhang der Akkommodation und der Vergenz messtechnisch abgebildet werden.

Gemäß Schritt a) des vorliegenden Verfahrens erfolgt eine Darstellung mindestens eines Zeichens in mindestens einer ersten Entfernung vor einem Auge eines Nutzers zur Stimulation der Akkommodation des Auges. Der Begriff des "Zeichens" betrifft hierbei Sehzeichen, insbesondere Buchstaben, Zahlen oder Symbole; Bilder oder Muster, welche farbig oder in schwarz-weiß dargestellt werden können. Während es sich bei den "Sehzeichen" jeweils um ein einzelnes feststehendes Zeichen handelt, das in seinen Proportionen zur Wiedererkennbarkeit durch den Nutzer nur eingeschränkt veränderbar ist, bezeichnet der Begriff des "Musters" eine beliebige graphische Struktur, welche - insbesondere im Gegensatz zu einem Rauschen, das ohne erkennbare Struktur bleibt - über mindestens eine räumlich orientierte Periode verfügt, innerhalb der die Struktur des Musters bevorzugt wiederholt dargestellt ist. Anstelle des Begriffs des "Musters" kann daher dann auch der Begriff des "periodischen Musters" verwendet werden, um diese Eigenschaft des Musters deutlich zum Ausdruck zu bringen, wobei hierin beide Begriffe denselben Begriffsinhalt umfassen.

Die Darstellung des mindestens einen Zeichens kann monokular jeweils getrennt für ein Auge erfolgen. Alternativ kann die Darstellung des mindestens einen Zeichens binokular gemeinsam und simultan für beide Augen eines Augenpaars erfolgen. Die Darstellung des mindestens einen Zeichens kann hierbei auf verschiedene Weise erfolgen, insbesondere auf einem Bildschirm, der in einer festen, aber wählbaren Entfernung vor dem mindestens einen Auge des Nutzers angeordnet sein kann. Der Begriff "Bildschirm" bezeichnet hierbei eine elektronisch ansteuerbare Anzeige, welche über eine zweidimensionale Ausdehnung verfügt, wobei das gewünschte mindestens eine Zeichen mit weitgehend frei wählbaren Parametern an einer beliebigen Stelle innerhalb der Ausdehnung darstellbar ist. Der Bildschirm kann hierbei bevorzugt ausgewählt sein aus einen Monitor, einem Screen oder einem Display, wobei der Bildschirm von der Auswerteeinheit angesteuert werden kann. Der Bildschirm kann hierbei für Aufblick oder, vorzugsweise, für Durchblick eingerichtet sein. Vorzugsweise kann der Bildschirm hierbei von einen mobilen Kommunikationsgerät umfasst sein. Der Begriff des "mobilen Kommunikationsgeräts" umfasst hierbei insbesondere ein Mobiltelefon (Handy), ein Smartphone oder ein Tablet. Andere Arten von mobilen Kommunikationsgeräten sind jedoch denkbar. Auf diese Weise kann das vorliegende Verfahren zur gemeinsamen Bestimmung von Akkommodation und Vergenz eines Auges oder beider Augen eines Nutzers an einem beliebigen Ort durchgeführt werden. Andere Arten von Bildschirmen sind jedoch ebenfalls möglich.

In einer alternativen Ausgestaltung kann das mindestens eine Zeichen mittels einer Projektionseinrichtung dargestellt werden. Hierbei kann die Projektionseinrichtung dazu eingerichtet sein, das mindestens eine Zeichen an eine vorbestimmte Stelle im Raum, die der festen, aber wählbaren Entfernung vor dem mindestens einen Auge des Nutzers entspricht, zu projizieren, wobei die Projektionseinrichtung von der Auswerteeinheit angesteuert werden kann. Alternativ oder zusätzlich kann das mindestens eine Zeichen derart auf das mindestens eine Auge des Augenpaars projiziert werden, dass der Nutzer das mindestens eine Zeichen virtuell an der vorbestimmten Stelle im Raum, welcher der festen, aber wählbaren Entfernung vor dem mindestens einen Auge des Nutzers entspricht, erkennen kann. Weitere Ausgestaltungen zur Darstellung des mindestens einen Zeichens in der gewünschten Entfernung vor dem mindestens einen Auge des Nutzers sind jedoch denkbar.

Aufgrund einer elektronischen Ansteuerung, insbesondere unter Verwendung der Auswerteeinheit, kann ein Parameter des dargestellten mindestens einen Zeichens auf einfache Weise und in einem weiten Rahmen verändert werden. Bei dem "Parameter" kann es sich um eine Eigenschaft des mindesten einen Zeichens, je nach ausgewähltem Zeichen, insbesondere um eine Ausdehnung, eine Orientierung, eine Position, eine Frequenz, einen Kontrast oder eine Farbe (einschließlich schwarz und weiß) handeln. Im Falle des Musters kann eine Struktur wiederholt dargestellt werden, wobei sich durch Wiederholung gleichartige Punkte oder Bereiche über die Struktur des Musters ausbilden können. Bevorzugte Ausgestaltungen gleichartiger Punkte oder Bereiche können bevorzugt als periodische Maxima oder Minima des Musters vorliegen. Während es sich bei den ausgewählten Parametern eines herkömmlichen Sehzeichens, insbesondere einem Buchstaben, einer Zahl oder einem Symbol, also um eine Ausdehnung, insbesondere eine Höhe oder Breite, des Zeichens handeln kann, bezieht sich bei dem periodischen Muster der Parameter bevorzugt auf einen Parameter einer periodischen Funktion, insbesondere eine Wiederholfrequenz. Die "periodische Funktion" bezeichnet hierbei eine Anweisung an eine Ausgestaltung einer zeitlich oder bevorzugt räumlich wiederholten Veränderung des Musters. Die periodische Funktion kann vorzugsweise ausgewählt sein aus einer Sinusfunktion, einer Cosinusfunktion oder einer Überlagerung hiervon. Andere periodische Funktionen sind jedoch denkbar.

In einer bevorzugten Ausgestaltung kann das dargestellte mindestens eine Zeichen ein Muster sein, wobei der zugehörige Parameter des Musters mindestens eine Ortsfrequenz des periodischen Musters umfasst. Der Begriff der "Ortsfrequenz" bezeichnet hierbei einen Kehrwert eines räumlichen Abstands, welcher in der Einheit 1/m oder insbesondere bei Kenntnis einer Entfernung von dem mindestens einen Auge des Nutzers alternativ oder zusätzlich auch als dimensionslose Zahl, zum Beispiel pro Grad oder pro Zyklus, zwischen zwei benachbart angeordneten gleichartigen Punkten, insbesondere einem Maximum oder einem Minimum, in einer räumlich periodischen Änderung des Musters angegeben werden kann. Andere Arten der Bestimmung der Ortsfrequenz aus dem Muster sind jedoch denkbar, beispielsweise aus einem Abstand von Punkten gleicher Intensität.

In dieser bevorzugten Ausgestaltung kann das periodische Muster als zweidimensionale Überlagerung einer periodischen Funktion, insbesondere der Sinusfunktion, welche sich in eine erste Richtung erstrecken kann, und einer konstanten Funktion, welche sich in eine zweite Richtung erstrecken kann, die vorzugsweise senkrecht zur ersten Richtung angeordnet sein kann, ausgestaltet sein. Der Begriff "senkrecht" bezeichnet hierbei einen Winkel von 90° ± 30°, bevorzugt von 90° ± 15°, besonders bevorzugt von 90° ± 5°, insbesondere von 90° ± 1°, jeweils zur ersten Richtung. Andere Winkel zwischen der ersten Richtung und der zweiten Richtung sind jedoch ebenfalls möglich. Auf diese Weise kann das Muster in Form von periodisch nebeneinander angeordneten Streifen vorliegen, die auch als "sinusförmiges Gitter" oder "Gabor Patch" bezeichnet werden können. Der Begriff "Gabor Patches" bezeichnet sinusförmige Gitter, die üblicherweise mit einer Gauß-förmigen Hülle versehen sind, und die dafür bekannt sind, dass sie sich insbesondere als Stimulus für mindestens ein Auge des Nutzers verwenden lassen. Andere Arten von Mustern sind jedoch möglich.

Gemäß Schritt b) erfolgt ein Erfassen einer Augenbewegung des mindestens einen Auges. Erfindungsgemäß dient das Erfassen einer Augenbewegung, bevorzugt mindestens einer ausgewählten Augenbewegungsmetrik, insbesondere unter Verwendung der Auswerteeinheit, dazu, die gesuchte Vergenz zu bestimmen, indem
- gegensinnige Augenbewegungen der beiden Augen eines Augenpaars des Nutzers, wobei jedes der beiden Augen eine Augenrotation um zueinander parallele Achsen, welche jeweils eine Verlängerung ins Unendliche der Verbindungslinie zwischen dem Zentrum der Pupille eines Auges und dessen Augendrehpunkt darstellen, in eine jeweils entgegengesetzte Rotationsrichtung sowohl zur Mittellinie als auch divergent weg von dieser ausführt, oder
- die Augenbewegung mindestens eines Auges des Nutzers von der Achse, welche die Verlängerung der Verbindungslinie ins Unendliche zwischen dem Zentrum der Pupille eines Auges und dessen Augendrehpunkt darstellt, sowohl zur Mittellinie als auch divergent weg von dieser, oder
- Augenbewegungen der beiden Augen eines Augenpaars des Nutzers, wobei jedes der beiden Augen unabhängig voneinander eine Augenrotation um seine jeweilige Achse, welche die Verlängerung ins Unendliche der Verbindungslinie zwischen dem Zentrum der Pupille eines Auges und dessen Augendrehpunkt darstellt, sowohl zur Mittellinie als auch divergent weg von dieser
jeweils erfasst und ausgewertet werden. Der Augendrehpunkt ist das geometrische Rotationszentrum des Auges. Die Mittellinie ist die senkrechte Projektion ins Unendliche in halber Pupillendistanz senkrecht zur Strecke der Pupillendistanz. In allen vorstehend genannten Ausführungen zur Vergenz und Divergenz beider Augen kann der Betrag der Augenrotation der beiden Augen unterschiedlich ausgeprägt sein. Diese unterschiedliche Ausprägung kann sowohl dann vorliegen, wenn die Augenrotation beider Augen vergent oder divergent ist als auch dann, wenn die Augenrotation eines der beiden Augen vergent und die Augenrotation des anderen Auges divergent ist.

In einer bevorzugten Ausgestaltung der vorliegenden Erfindung kann, wie unten näher erläutert, das Erfassen einer Augenbewegung darüber hinaus auch zur Ermittlung der Refraktion des mindestens einen Auges gemäß Schritt c) dienen, wozu beispielsweise das in der europäischen Patentanmeldung Az. 19 170 561.5, angemeldet am 23.04.2019, offenbarte Verfahren oder bevorzugt wenigstens eine Refraktionsmesseinrichtung ausgewählt aus der Gruppe umfassend wenigstens ein Shack-Hartmann Aberrometer, wenigstens ein exzentrischen Photorefraktor und wenigstens ein Auto-Focus-System, eingesetzt werden kann. Die Refraktionsmesseinrichtung umfasst besonders bevorzugt wenigstens ein Shack-Hartmann Aberrometer. Hierbei kann ein Shack-Hartmann Aberrometer als optischer Sensor verwendet werden, welcher beispielsweise wenigstens ein Mikrolinsenarray und wenigstens einen Kamerasensor umfassen kann und zur Erfassung der Refraktion sowie Fehlern höherer Ordnung des mindestens einen Auges dient. Ein exzentrischer Photorefraktor dient ebenfalls als optischer Sensor zur Erfassung wenigstens eines Lichtreflexes, wobei der Photorefraktor wenigstens einen Kamerasensor und optional eine exzentrisch angeordnete Lichtquelle, beispielsweise eine Infrarotlichtquelle, umfassen kann. Bevorzugt umfasst der Photorefraktor wenigstens einen Kamerasensor und eine exzentrisch angeordnete Lichtquelle. Aus dem wenigstens einen Lichtreflex kann die Refraktion des mindestens einen Auges berechnet werden. Für die Erzeugung des wenigstens einen Lichtreflexes muss die Lichtquelle nicht Bestandteil des exzentrischer Photorefraktors sein, sondern kann von diesem unabhängig angeordnet sein. Ein Auto-Focus-System dient auch als optischer Sensor zur Erfassung der Refraktion des mindestens einen Auges, wobei die Refraktion durch die Optimierung mindestens einer optischen Qualität, bevorzugt der Bildschärfe und/oder des Bildkontrastes, auf wenigstens einem Kamerasensor bestimmt wird. Die Optimierung erfolgt bevorzugt mittels unterschiedlicher optischer Fokussierung und Auswertung der optischen Qualität der zu der jeweiligen optischen Fokussierung durch den wenigstens einen Kamerasensor erfassten Kameraaufnahmen. Die wenigstens eine Refraktionsmesseinrichtung kann weiterhin wenigstens eine Linse und/oder wenigstens eine Blende umfassen. Die wenigstens eine Refraktionsmesseinrichtung umfasst bevorzugt wenigstens einen exzentrischen Photorefraktor oder wenigstens ein Auto-Focus-System kann beispielsweise Bestandteil wenigstens eines mobilen Endgeräts oder mit wenigstens einem mobilen Endgerät verbunden sein, beispielsweise in Form i) wenigstens einer Kamera oder ii) wenigstens einer Lichtquelle und wenigstens einer Kamera oder wenigstens eines Lichtempfängers, jeweils des wenigstens einen mobilen Endgeräts.

Der Begriff "Augenbewegungsmetrik" bezeichnet hierbei ein Maß, welches mit einer Bewegung des mindestens einen Auges des Nutzers verknüpft ist, wobei die Bewegung des mindestens einen Auges des Nutzers von dem äußeren, auf das mindestens eine Auge des Nutzers einwirkenden Stimulus in Form mindestens eines Zeichens hervorgerufen wird. Im Rahmen der vorliegenden Erfindung kann sich die Augenbewegungsmetrik vorzugsweise beziehen auf: gegensinnige Augenbewegungen der beiden Augen eines Augenpaars des Nutzers, wobei jedes der beiden Augen eine Augenrotation um zueinander parallele Achsen, welche jeweils eine Verlängerung ins Unendliche der Verbindungslinie zwischen dem Zentrum der Pupille eines Auges und dessen Augendrehpunkt darstellen, in eine jeweils entgegengesetzte Rotationsrichtung sowohl zur Mittellinie als auch divergent weg von dieser ausführt; Augenbewegungen mindestens eines Auges des Nutzers von der Achse, welche die Verlängerung der Verbindungslinie ins Unendliche zwischen dem Zentrum der Pupille eines Auges und dessen Augendrehpunkt darstellt, sowohl zur Mittellinie als auch divergent weg von dieser; Augenbewegungen der beiden Augen eines Augenpaars des Nutzers, wobei jedes der beiden Augen unabhängig voneinander eine Augenrotation um seine jeweilige Achse, welche die Verlängerung ins Unendliche der Verbindungslinie zwischen dem Zentrum der Pupille eines Auges und dessen Augendrehpunkt darstellt, sowohl zur Mittellinie als auch divergent weg von dieser; eine Augenfolgebewegung; eine Augenbewegung, die sich auf Mikrosakkaden bezieht, die eine Mikrosakkadenrichtung, eine Mikrosakkadenrate oder eine Sakkadengenauigkeit umfasst; oder einen optokinetischen Nystagmus. Weitere Augenbewegungsmetriken können zum Beispiel eine Verweildauer beim flüssigen Lesen des dargestellten mindestens einen Zeichens, die auch als "Fixationsdauer" bezeichnet wird, umfassen. Weitere Arten an Augenbewegungen können darüber hinaus ebenfalls erfasst werden. Welche Art von Augenbewegungsmetrik oder welche Kombination von mindestens zwei Augenbewegungsmetriken herangezogen werden, hängt im Wesentlichen ab von einer Genauigkeit einer hierzu verwendeten Einrichtung sowie von dem jeweiligen Verwendungszweck. Während sich gegensinnige Augenbewegungen der beiden Augen eines Augenpaars des Nutzers, wobei jedes der beiden Augen eine Augenrotation um zueinander parallele Achsen, welche jeweils eine Verlängerung ins Unendliche der Verbindungslinie zwischen dem Zentrum der Pupille eines Auges und dessen Augendrehpunkt darstellen, in eine jeweils entgegengesetzte Rotationsrichtung sowohl zur Mittellinie als auch divergent weg von dieser, Augenbewegungen mindestens eines Auges des Nutzers von der Achse, welche die Verlängerung der Verbindungslinie ins Unendliche zwischen dem Zentrum der Pupille eines Auges und dessen Augendrehpunkt darstellt, sowohl zur Mittellinie als auch divergent weg von dieser oder voneinander unabhängige Augenbewegungen der beiden Augen eines Augenpaars des Nutzers, wobei jedes der beiden Augen unabhängig voneinander eine Augenrotation um seine jeweilige Achse, welche die Verlängerung ins Unendliche der Verbindungslinie zwischen dem Zentrum der Pupille eines Auges und dessen Augendrehpunkt darstellt, sowohl zur Mittellinie als auch divergent weg von dieser jeweils insbesondere zur Bestimmung der Vergenz verwenden lassen, können sich Augenfolgebewegungen bevorzugt zur Bestimmung der Refraktion eignen.

Der Begriff "Augenfolgebewegung" bezeichnet hierbei eine Bewegung des mindestens einen Auges, durch welche das mindestens eine Auge den Bewegungen des dargestellten Zeichens, das von dem mindestens einen Auge fixiert wird, nachfolgt. Im Allgemeinen handelt es sich bei der Augenfolgebewegung um eine langsame Bewegung des mindestens einen Auges mit einer Winkelgeschwindigkeit von 0,5 °/s bis 50 °/s, bei welcher ein Abbild des Zeichens vorzugsweise auf der Fovea des mindestens einen Auges verbleibt. Die Augenfolgebewegungen können nicht willkürlich erzeugt werden, sondern setzen voraus, dass das dargestellte Zeichen eine Bewegung ausführt, welcher das mindestens eine Auge des Nutzers folgen kann.

Augenbewegungsmetriken, die sich auf Sakkaden oder Mikrosakkaden beziehen, können im Rahmen der vorliegenden Erfindung vorzugsweise als Maß dafür verwendet werden, um festzustellen, ob der Nutzer das dargestellte Zeichen als Stimulus erkannt hat oder nicht. Der Begriff "Sakkade" bezeichnet ruckartige Blickzielbewegungen des mindestens einen Auges des Nutzers, die zielbezogen ausgeführt werden, die eine geringe Amplitude von mindestens 1° aufweisen und die insbesondere zu einer schnellen regelmäßigen Neuausrichtung einer Blickrichtung des mindestens einen Auges auf einen Fixationspunkt dienen, vorzugsweise indem das Abbild eines sich in dem Fixationspunkt befindlichen Zeichens aus einer Peripherie auf die Fovea des mindestens einen Auges verschoben wird. Eine "Sakkadenrate" beträgt typischerweise 1 Hz bis 5 Hz, wobei eine Winkelgeschwindigkeit von 5 °/s bis 500 °/s erreicht werden kann. Der Begriff "Mikrosakkade" bezeichnet kleine ruckartige und unwillkürliche Blickbewegungen, die nicht auf ein Ziel bezogen sein können, die zufällig auftreten und deren Amplitude geringer als 1° ist. Die "Mikrosakkadenrichtung" betrifft eine räumliche Orientierung der Mikrosakkade relativ zu einem Koordinatensystem, vorzugsweise einem durch das dargestellte Zeichen festgelegten Koordinatensystem. Hierbei kann die Orientierung relativ zu dem dargestellten Zeichen als Maß des Erkennens dienen. Die "Sakkadengenauigkeit" bezeichnet eine räumliche Präzision einer Neuausrichtung relativ zu einer neuen Position eines Stimulus. Kann der Stimulus nach der Neuausrichtung schlechter wahrgenommen werden, ist hierbei ein zu erwartender Fehler der Neuausrichtung größer.

Alternativ oder zusätzlich können Augenbewegungsmetriken, welche sich auf den optokinetischen Nystagmus beziehen, vorzugsweise als Maß dafür verwendet werden, um festzustellen, ob der Nutzer das dargestellte Zeichen als Stimulus erkannt hat oder nicht. Der Begriff "optokinetischer Nystagmus" bezeichnet einen physiologischen Augenbewegungsreflex, welcher durch eine langsame und eine schnelle Phase charakterisiert ist. Hierbei entspricht die langsame Phase einer Folgebewegung der Geschwindigkeit eines bewegten Stimulus' der Umgebung. Eine Korrelation der Phase oder der Geschwindigkeit des Stimulus' mit der langsamen Phase des optokinetischen Nystagmus kann als Maß dafür verwendet werden, ob ein Nutzer den Stimulus erkennt. Des Weiteren ist denkbar, eine Korrelation der Phase oder der Geschwindigkeit des Stimulus' mit der schnellen Phase des optokinetischen Nystagmus als Maß dafür zu verwenden, ob ein Nutzer den Stimulus erkennt.

Zur Erfassung der Augenbewegungsmetriken kann eine Augenbewegungsmesseinrichtung, die auch als "Eyetracker" bezeichnet wird, eingesetzt und, insbesondere unter Verwendung der Auswerteeinheit, angesteuert werden. Die Augenbewegungsmesseinrichtung kann vorzugsweise eine Kamera, besonders bevorzugt eine Videokamera, umfassen, insbesondere um ein videobasiertes "Eyetracking" durchführen zu können, indem vorzugsweise Bildfolgen einer Augenpartie des Nutzers aufgenommen und mittels Bildverarbeitung ausgewertet werden, um hieraus mindestens eine der Augenbewegungsmetriken zu ermitteln. Hierfür lassen sich insbesondere jeweils bekannte Algorithmen einsetzen. Darüber hinaus können aus den aufgenommenen Bildfolgen mittels Bildverarbeitung, insbesondere unter Verwendung der Auswerteeinheit, weiterhin geometrische Daten des mindestens einen Auges, bevorzugt dessen Pupille, insbesondere Position und Durchmesser dessen Pupille, ermittelt werden und hieraus zum Beispiel die Blickrichtung des mindestens einen Auges bestimmt werden. Hierzu sind, insbesondere unter Verwendung der Auswerteeinheit, Verfahren einsetzbar, welche ausgewählte Reflexionspunkte einbeziehen, die auf Vorderseite und/oder Rückseite von Cornea und Linse entstehen können, wenn das mindestens eine Auge durch eine Lichtquelle angestrahlt wird. Insbesondere kann eine Blickrichtung aus Hornhautreflex und Pupillenposition bestimmt werden, siehe zum Beispiel P. Blignaut, Mapping the Pupil-Glint Vector to Gaze Coordinates in a Simple Video-Based Eye Tracker, Journal of Eye Movement Research 7(1):4, Seite 1-11, 1995. Grundsätzlich können aber auch andere Reflexe aufgenommen werden, insbesondere mittels eines so genannten "Dual Purkinje Eyetrackers". Da sich der Hornhautreflex ohne Kopfbewegung nicht bewegt, jedoch die Pupille ihre Position bei der Augenbewegung ändert, kann hieraus auf die Augenrotation geschlossen werden. Die "Pupille" bezeichnet hierbei eine in jedem Auge vorhandene Eintrittsöffnung, durch welche Strahlung in Form von Licht in das Innere des Auges eintreten kann. In umgekehrter Richtung kann die Pupille als Austrittsöffnung betrachtet werden, durch welche die Blickrichtung des Nutzers aus dem Auge auf die Umgebung festgelegt werden kann.

Weiterhin kann eine Beleuchtungseinrichtung vorgesehen sein, insbesondere um die Augenbewegungsmetrik des Nutzers mit möglichst hoher Auflösung und möglichst hohem Kontrast mittels der Kamera, insbesondere der Videokamera, erfassen zu können. Alternativ oder zusätzlich kann auf Tageslicht oder auf eine bereits vorhandene Beleuchtung zurückgegriffen werden. Die Beleuchtungseinrichtung kann hierbei als Lichtquelle ausgestaltet sein, die von der Augenbewegungsmesseinrichtung umfasst oder als gesonderte Einrichtung eingerichtet sein kann.

In einer besonderen Ausgestaltung kann die Kamera, insbesondere die Videokamera, eine Sensitivität im infraroten Spektralbereich, d.h. bei einer Wellenlänge von 780 nm bis 1 mm, bevorzugt von 780 nm bis 3 µm, insbesondere von 780 nm bis 1,4 µm (nach DIN EN ISO 13666:2013-10, Abschnitt 4.4, auch als "IR-A" bezeichnet), aufweisen. Um Infrarotstrahlung bereitzustellen, kann die hierfür vorgesehene Lichtquelle im infraroten Spektralbereich abstrahlen insbesondere mit einer Wellenlänge, für welche die Kamera eine ausreichende Sensitivität aufweist. Die Lichtquelle kann vorzugsweise ausgewählt sein aus einer Mikroglühlampe, einem IR-Emitter auf Festkörperbasis, einer lichtemittierenden Diode oder einem Infrarotlaser, wobei entsprechende Filter verwendet werden können.

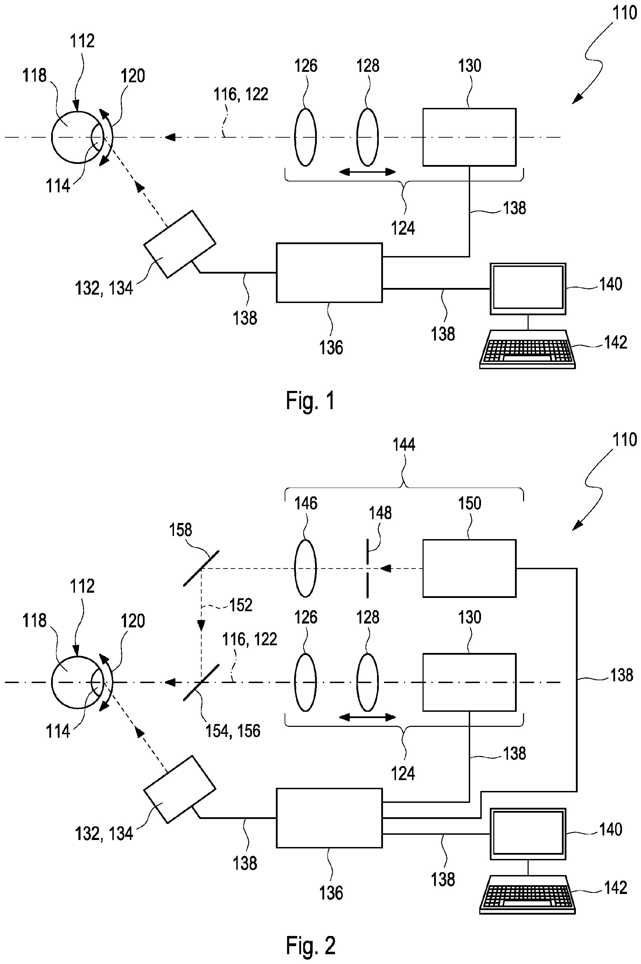

Gemäß Schritt c) erfolgt ein Ermitteln einer Refraktion des mindestens einen Auges bei der Akkommodation des mindestens einen Auges in die mindestens eine erste Entfernung. Wie unten näher erläutert, kann die Refraktion des mindestens einen Auges mittels einer Refraktionsmesseinrichtung, die zur Erfassung der Refraktion des mindestens einen Auges eingerichtet ist, ermittelt werden, wobei die Ansteuerung der Refraktionsmesseinrichtung insbesondere unter Verwendung der Auswerteeinheit erfolgen kann. Die Refraktionsmesseinrichtung kann hierbei, wie z.B. in US 2012/0287398 A1 offenbart, eine Anzahl an optischen Elementen umfassen, welche zur Bestimmung der Defokussierung des mindestens einen Auges des Nutzers eingerichtet sind. Eine bevorzugte Ausgestaltung der Refraktionsmesseinrichtung ist in den Ausführungsbeispielen unter Bezugnahme auf die Figur 2 dargestellt. Andere Arten der Ausgestaltung der Refraktionsmesseinrichtung sind jedoch möglich.

In einer alternativen Ausgestaltung, kann die Refraktion des Auges mittels einer Erfassung der Augenbewegung des mindestens einen Auges des Nutzers, vorzugsweise während gemäß Schritt b), erfolgen. Wie in der europäischen Patentanmeldung Az. 19 170 561.5, angemeldet am 23.04.2019, offenbart, kann das Erfassen der Augenbewegung des mindestens einen Auges des Nutzers, insbesondere unter Verwendung der Auswerteeinheit, in Abhängigkeit von dem Zeichen erfolgen, während ein Parameter des Zeichens verändert wird, wobei ein Zeitpunkt festgestellt werden kann, zu dem sich aus der Augenbewegung eine Erkennungsschwelle des Nutzers für das Zeichen ergibt, wobei sich die Refraktion des mindestens einen Auges aus dem zu dem Zeitpunkt festgelegten Parameter für das Zeichen ermitteln lässt. Das Erfassen der Augenbewegung hierbei kann für verschiedene Werte des Parameters wiederholt werden, vorzugsweise solange bis der gewünschte Zeitpunkt festgestellt wurde. Hierbei bezeichnet der Begriff "Erkennungsschwelle", dass der Nutzer das dargestellte Zeichen als Stimulus für das mindestens eine Auge gerade noch oder gerade erst wahrnehmen kann. Nimmt einer der Parameter des Zeichens, insbesondere die Ortsfrequenz in dem periodischen Muster, zunehmend zu, kann hierbei der Zeitpunkt festgestellt werden, an dem das Zeichen gerade nicht mehr als Stimulus für das mindestens eine Auge des Nutzers wirken kann. Umgekehrt kann, falls einer der Parameter des Zeichens, insbesondere die Ortsfrequenz in dem periodischen Muster, zunehmend abnimmt, hierbei der Zeitpunkt festgestellt werden, an dem das dargestellte Zeichen erstmals nicht als Stimulus für das mindestens eine Auge des Nutzers wirken kann. Alternativ kann auch beispielsweise, wenn einer der Parameter des Zeichens, insbesondere die Ortsfrequenz in dem periodischen Muster, zunehmend abnimmt, hierbei der Zeitpunkt festgestellt werden, an dem das dargestellte Zeichen gerade als Stimulus für das mindestens eine Auge des Nutzers wirken kann. Umgekehrt kann in diesem Fall dann, falls einer der Parameter des Zeichens, insbesondere die Ortsfrequenz in dem periodischen Muster, zunehmend zunimmt, hierbei der Zeitpunkt festgestellt werden, an dem das dargestellte Zeichen erstmals als Stimulus für das mindestens eine Auge des Nutzers wirken kann.

In einer besonderen Ausgestaltung der vorliegenden Erfindung kann der Zeitpunkt, zu dem sich aus der Reaktion des Nutzers die Erkennungsschwelle des Nutzers für das dargestellte Zeichen ergibt, dadurch festgestellt werden, dass die Augenbewegungsmetrik des Nutzers der Bewegung des dargestellten Zeichens gerade noch folgt oder gerade erst beginnt zu folgen. Insbesondere kann hierzu die Augenfolgebewegung des Nutzers, mit welcher er den Bewegungen eines Zeichens, das von dem mindestens einen Auge fixiert wird, nachfolgt, zur Feststellung des gewünschten Zeitpunkts verwendet werden, insbesondere da, wie oben erwähnt, die Augenfolgebewegungen nicht willkürlich erzeugt werden können, sondern der Bewegung des dargestellten Zeichens, das als Stimulus für das mindestens eine Auge des Nutzers dient, folgen. Hierbei kann der gewünschte Zeitpunkt, zu dem sich aus der Augenbewegungsmetrik des Nutzers die Erkennungsschwelle des Nutzers für das dargestellte Zeichen, insbesondere die Ortsfrequenz des Musters, festgestellt werden. Hierzu können bevorzugt Daten zur Erfassung der Augenbewegung des Nutzers, welche die Videokamera aufgenommen hat, dazu verwendet werden, um die Blickrichtung des Nutzers auf das dargestellte Zeichen zu ermitteln. Im Falle einer zunehmenden Abnahme eines der Parameter des Zeichens, insbesondere der Ortsfrequenz in dem periodischen Muster, wird die Augenfolgebewegung des Nutzers solange der Bewegung des Zeichens entsprechen, solange der Nutzer das dargestellte Zeichen erkennen kann. Ist ein Zeitpunkt erreicht, zu dem der Nutzer das dargestellte Zeichen, insbesondere das periodische Muster, gerade nicht mehr erkennen kann und das somit nicht mehr als Stimulus für das mindestens eine Auge des Nutzers wirken kann, so weicht die Augenfolgebewegung des Nutzers von der Bewegung des Zeichens ab. Wird umgekehrt der Zeitpunkt erreicht, zu dem der Nutzer das dargestellte Zeichen, insbesondere das periodische Muster, erstmals gerade erkennen kann und das somit erstmals als Stimulus für das mindestens eine Auge des Nutzers wirken kann, so wird die Augenfolgebewegung des Nutzers nun beginnen, der Bewegung des Zeichens zu folgen. Unabhängig von der Art der Ausgestaltung kann hierbei bevorzugt eine Schwelle festgesetzt werden, mittels der ein Grad der Abweichung der Augenfolgebewegung des Nutzers von der Bewegung des Zeichens als der gesuchte Zeitpunkt festgelegt wird. Der Zeitpunkt, zu dem die Abweichung die festgelegte Schwelle überschreitet oder unterschreitet, stellt hierbei den gesuchten Zeitpunkt dar.

Vorzugsweise im Anschluss an die Feststellung des Zeitpunkts kann die Bestimmung eines Wertes für die Refraktion des mindestens einen Auges des Nutzers aus dem Wert des Parameters erfolgen, der zu dem festgestellten Zeitpunkt dazu verwendet wurde, den ausgewählten Parameter des Zeichens einzustellen. In der oben beschriebenen Ausgestaltung kann hierbei der Wert für die Refraktion aus der zu dem Zeitpunkt festgelegten Ortsfrequenz des Musters, welche auch eine Grenzfrequenz des Musters sein kann, bestimmt werden, zu der sich aus der Beobachtung der Augenbewegungsmetrik des Nutzers die Erkennungsschwelle des Nutzers für das dargestellte Zeichen ergibt. Die "Grenzfrequenz" bezeichnet diejenige Ortsfrequenz des Musters, bei welcher die Kontrastempfindlichkeit Null wird oder der Kontrast des Stimulus maximal. Diese Frequenz kann auch als Auflösungsgrenze des visuellen Systems betrachtet werden. Der Begriff der "Kontrastempfindlichkeit" des mindestens einen Auges definiert hierbei ein Maß zur Unterscheidung verschiedener Grauabstufungen als Kehrwert des kleinsten, gerade noch wahrnehmbaren Unterschiedes zweier Grauwerte. Die Begriffe "Sehschärfe" und "Auflösungsvermögen" des mindestens einen Auges des Nutzers geben hierbei jeweils ein Maß für einen räumlichen Abstand zwischen zwei Punkten, den das mindestens eine Auge des Nutzers noch als unterscheidbar wahrnehmen kann. In der oben beschriebenen Ausgestaltung kann die Kontrastempfindlichkeit mittels eines periodischen Musters in Form von periodisch nebeneinander angeordneten Streifen, das auch als "sinusförmiges Gitter" oder als "Gabor Patch" bezeichnet wird, ermittelt werden. Wie in A. Leube et al., Individual neural transfer function affects the prediction of subjective depth offocus, Scientific Reports 2018, 8(1), 1919 beschrieben, werden hierzu bevorzugt Gabor Patches verwendet, deren Kontrast so lang verringert wird, bis kein Streifenmuster mehr wahrgenommen wird. Dieser Kontrastwert wird als Erkennungsschwelle herangezogen. Dieser Vorgang wird für verschiede Ortsfrequenzen wiederholt. Für weitere Einzelheiten wird auf die Ausführungsbeispiele verwiesen.

Unabhängig von der Art der Ermittlung der bei dem Nutzer auftretenden Refraktion kann hieraus eine sphärozylindrische Linse ermittelt werden, welche als Brillenglas dazu benutzt werden kann, den eine Defokussierung hervorrufenden Refraktionsfehler des mindestens einen Auges derart zu kompensieren, dass sich eine möglichst optimale Bildqualität für den Nutzer erzielen lässt. Zur Beschreibung der sphärozylindrischen Linse eignen sich unterschiedliche Darstellungsweisen. Die Norm DIN EN ISO 13666:2013-10, im Folgenden auch als "Norm" bezeichnet, legt in Abschnitt 11.2 einen so genannten "sphärischen Brechwert" fest, welcher als Wert für einen Scheitelbrechwert eines Brillenglases mit sphärischer Wirkung oder für den jeweiligen Scheitelbrechwert in einem von zwei Hauptschnitten des Brillenglases mit astigmatischer Wirkung definiert ist. Nach der Norm, 9.7.1 und 9.7.2 ist der "Scheitelbrechwert" als Kehrwert einer paraxialen Schnittweite eines bildseitigen Brennpunktes, jeweils gemessen in Metern, festgelegt. Das sphärozylindrische Brillenglas mit astigmatischer Wirkung vereinigt gemäß der Norm, Abschnitt 12, ein paraxiales, paralleles Lichtbündel in zwei getrennten, zueinander senkrecht stehenden Brennlinien, und besitzt daher nur in den beiden Hauptschnitten einen sphärischen Scheitelbrechwert. Die "astigmatische Wirkung" ist hierbei durch Zylinderstärke und Achslage festgelegt. Hierbei stellt die "Zylinderstärke" gemäß der Norm, 12.5 den Betrag einer "astigmatischen Differenz" dar, welche die Differenz zwischen den Scheitelbrechwerten in den beiden Hauptschnitten angibt. Die "Achslage" bezeichnet gemäß der Norm, 12.6 eine Richtung des Hauptschnittes, dessen Scheitelbrechwert als Referenzwert herangezogen wird. Schließlich wird nach der Norm, 12.8 die "Stärke" des Brillenglases mit astigmatischer Wirkung mittels drei Werten, umfassend die Scheitelbrechwerte jedes der beiden Hauptschnitte und die Zylinderstärke, angegeben. Weiterhin kann die Beschreibung der sphärozylindrischen Linse durch Angabe eines "Refraktionsvektors" (*power vector*) nach L. N. Thibos, W. Wheeler und D. Horner (1997), Power Vectors: An Application of Fourier Analysis to the Description and Statistical Analysis of Refractive Error, Optometry and Vision Science 74 (6), S. 367-375, erfolgen. Der Refraktionsvektor, welcher durch genau einen Punkt in einem dreidimensionalen dioptrischen Raum beschrieben werden kann, wobei der dreidimensionale dioptrische Raum durch Koordinaten aufgespannt werden kann, entspricht bzw. korreliert mit der mittleren sphärischen Refraktion und der Zylinderstärke sowie der dazugehörigen Achslage.

Gemäß Schritt d) erfolgt das gemeinsame Bestimmen der Akkommodation und der Vergenz des mindestens einen Auges, insbesondere unter Verwendung der Auswerteeinheit, einerseits durch ein Ermitteln einer Änderung der Refraktion des mindestens einen Auges bei der Akkommodation des mindestens einen Auges in die mindestens eine erste Entfernung gegenüber der Akkommodation des mindestens einen Auges in eine zweite Entfernung, und andererseits durch ein Ermitteln der Vergenz des mindestens einen Auges aus der Augenbewegung des mindestens einen Auges bei der Akkommodation des mindestens einen Auges in die mindestens eine erste Entfernung. Besonders bevorzugt kann hierbei die oben genannte Größe "AC/A" bestimmt werden, d.h. derjenige Betrag für die Vergenz, den das mindestens eine Auge selbsttätig für eine bestimmte Akkommodationsentfernung einstellt.

Erfindungsgemäß erfolgt somit einerseits das Ermitteln der Änderung der Refraktion des mindestens einen Auges bei dessen Akkommodation in die mindestens eine erste Entfernung gegenüber dessen Akkommodation in mindestens eine hiervon verschiedene zweite Entfernung. Hierzu erfolgt eine Veränderung einer Fixierung des mindestens einen Auges des Nutzers von einer zweiten Entfernung zu einer ersten Entfernung, wobei sich das gemäß Schritt a) dargestellte und auf die Netzhautebene des mindestens einen Auges möglichst scharf abzubildende Zeichen zunächst in der zweiten Entfernung und hieran anschließend in der ersten Entfernung befindet. Hierbei liegen sowohl die erste Entfernung als auch die zweite Entfernung zwischen dem Nahpunkt und dem Fernpunkt des mindestens einen Auges, wobei in einer bevorzugten Ausgestaltung die mindestens eine erste Entfernung für einen akkommodierten Zustand des mindestens einen Auges und die zweite Entfernung für einen akkommodationslosen Zustand des mindestens einen Auges ausgewählt werden kann.

Somit kann in einer besonders bevorzugten Ausführung des vorliegenden Verfahrens die Ermittlung der Refraktion des mindestens einen Auges des Nutzers bei mindestens zwei voneinander verschiedenen Entfernungen des dargestellten Zeichens vor dem mindestens einen Auge ermittelt werden.

Bevorzugt können für die mindestens zwei, bevorzugt mindestens drei, mindestens vier, mindestens fünf oder mindestens sechs, ersten oder zweiten Entfernungen einerseits Werte von 15 cm bis 60 cm, besonders bevorzugt von 20 cm bis 50 cm, insbesondere bei etwa 20 cm, 25 cm, 40 cm und 50 cm, bei welchen ein akkommodierter Zustand des mindestens einen Auges vorliegt, und andererseits ein Werte von mindestens 1 m, bevorzugt mindestens 1,5 m, besonders bevorzugt mindestens 2 m, vor dem mindestens einen Auge, das hierbei im Wesentlichen einen akkommodationslosen Zustand annimmt, ausgewählt werden. Eine andere Anzahl von Entfernungen, für die die Ermittlung der Refraktion durchgeführt wird, sowie andere Werte für die jeweils gewählten Entfernungen sind jedoch möglich. Durch die Ermittlung von Werten für die Refraktion bei zwei oder mehr voneinander verschiedenen ersten Entfernungen kann in vorteilhafter Weise eine Messkurve erhalten werden, aus welcher eine Auswertung der Änderung der Refraktion mit erhöhter Genauigkeit ermöglicht wird. Andere Arten der Ermittlung der Änderung der Refraktion bei einer der Änderung der ersten oder zweiten Entfernung sind jedoch denkbar.

In einer besonderen Ausgestaltung, die in bevorzugter Weise insbesondere bei einer Verlaufskontrolle der gemeinsamen Bestimmung von Akkommodation und Vergenz des mindestens einen Auges des Nutzers über einen Zeitraum, beispielsweise über eine oder mehrere Wochen, über wenigstens einen Monat, wenigstens ein Quartal oder wenigstens ein Jahr, zum Einsatz kommen kann, kann auf eine aktuelle Messung der Refraktion bei der Akkommodation des mindestens einen Auges in die zweite Entfernung verzichtet werden und stattdessen ein bekannter Wert für die Refraktion bei der Akkommodation des mindestens einen Auges in die zweite Entfernung, bevorzugt aus einer früheren Ermittlung dieses Wertes, verwendet werden, unabhängig davon, ob dieser Wert mit dem vorliegenden Verfahren oder mit einem anderen Verfahren ermittelt wurde. Hierzu kann eine frühere Bestimmung des Wertes für die Refraktion des mindestens einen Auges im akkommodationslosen Zustand mit einem üblichen Refraktometer, wie beispielsweise in einem Brillenpass für den Nutzer festgehalten, dienen. In dieser besonderen Ausgestaltung kann es somit genügen, aktuell mindestens einen Wert für die Refraktion bei der Akkommodation des mindestens einen Auges in die erste Entfernung messtechnisch zu erfassen und zur Ermittlung der Änderung der Refraktion des mindestens einen Auges während Schritt d) auf einen bekannten Wert für die Refraktion des mindestens einen Auges bei der Akkommodation in die zweite Entfernung zurückzugreifen.

Erfindungsgemäß erfolgt somit gemeinsam das Ermitteln der Vergenz des mindestens einen Auges aus der Augenbewegung des mindestens einen Auges bei der Akkommodation des mindestens einen Auges in die mindestens eine erste Entfernung. Wie oben bereits erwähnt, bezeichnet der Begriff der "Vergenz"
- gegensinnige Augenbewegungen der beiden Augen eines Augenpaars des Nutzers, wobei jedes der beiden Augen eine Augenrotation um zueinander parallele Achsen, welche jeweils eine Verlängerung ins Unendliche der Verbindungslinie zwischen dem Zentrum der Pupille eines Auges und dessen Augendrehpunkt darstellen, in eine jeweils entgegengesetzte Rotationsrichtung sowohl zur Mittellinie als auch divergent weg von dieser ausführt oder
- die Augenbewegung mindestens eines Auges des Nutzers von der Achse, welche die Verlängerung der Verbindungslinie ins Unendliche zwischen dem Zentrum der Pupille eines Auges und dessen Augendrehpunkt darstellt, sowohl zur Mittellinie als auch divergent weg von dieser, oder
- Augenbewegungen der beiden Augen eines Augenpaars des Nutzers, wobei jedes der beiden Augen unabhängig voneinander eine Augenrotation um seine jeweilige Achse, welche die Verlängerung ins Unendliche der Verbindungslinie zwischen dem Zentrum der Pupille eines Auges und dessen Augendrehpunkt darstellt, sowohl zur Mittellinie als auch divergent weg von dieser.

Vorzugsweise kann daher das Ermitteln der Vergenz des mindestens einen Auges mittels einer Erfassung einer Augenrotation des mindestens einen Auges während der Akkommodation des mindestens einen Auges in die mindestens eine erste Entfernung erfolgen. In einer besonders bevorzugten Ausgestaltung kann der Hornhautreflex der Pupille des betreffenden mindestens einen Auges aufgenommen werden. Dies ist insbesondere daher von Vorteil, weil sich der Hornhautreflex ohne Kopfbewegung nicht bewegt, jedoch die Pupille ihre Position bei der Augenbewegung ändert, so dass hieraus zuverlässig auf die Augenrotation geschlossen werden kann. Andere Arten der Ermittlung der Vergenz des mindestens einen Auges, insbesondere der Augenrotation, sind jedoch denkbar. Insbesondere kann eine Bewegung der Iris des betreffenden mindestens einen Auges oder den oben genannten "Dual Purkinje Eyetracker" verwendet werden.

In einem weiteren Aspekt betrifft die vorliegende Erfindung ein Computerprogramm, das ausführbare Anweisungen zur Durchführung des hierin beschriebenen Verfahren umfasst. Für Ausgestaltungen des Computerprogramms wird auf die Beschreibung des Verfahrens verwiesen.

In weiteren Aspekten betrifft die vorliegende Erfindung eine Vorrichtung zur Datenverarbeitung, einen computerlesbaren Datenträger, Datenträgersignale und ein computerlesbares Medium. Für Ausgestaltungen dieser Aspekte wird auf den jeweils zugehörigen Gegenstand der Datenverarbeitung, des Datenträgers, des Datenträgersignals oder des computerlesbaren Mediums verwiesen.

In einem weiteren Aspekt betrifft die vorliegende Erfindung eine Vorrichtung zur gemeinsamen Bestimmung von Akkommodation und Vergenz mindestens eines Auges eines Nutzers. Erfindungsgemäß umfasst die Vorrichtung
- eine Einrichtung, die zur Darstellung eines Zeichens in mindestens einer ersten Entfernung vor mindestens einem Auge eines Nutzers zur Stimulation der Akkommodation des mindestens einen Auges eingerichtet ist;
- eine Augenbewegungsmesseinrichtung, die zur Erfassung mindestens einer Augenbewegung des mindestens einen Auges eingerichtet ist; und
- eine Auswerteeinheit, die zur gemeinsamen Bestimmung der Akkommodation aus der Änderung der Refraktion und der Vergenz aus der Augenbewegung gemäß dem hierin beschriebenen Verfahren eingerichtet ist.

In einer bevorzugten Ausgestaltung kann die Vorrichtung eine an anderer Stelle hierin beschriebene Refraktionsmesseinrichtung umfassen, die zur Erfassung der Refraktion des mindestens einen Auges eingerichtet ist. Hierbei kann die Refraktionsmesseinrichtung bevorzugt ausgewählt sein aus der Gruppe umfassend wenigstens ein Shack-Hartmann Aberrometer, wenigstens einen exzentrischen Photorefraktor und wenigstens ein Auto-Focus-System. Für Ausführungsvarianten lassen sich hierbei verschiedene Wellenlängen und Strahlengänge verwenden. Alternativ oder zusätzlich kann die Erfassung der Refraktion des mindestens einen Auges mittels der Augenbewegungsmesseinrichtung erfolgen. Hierzu wird auf die entsprechende Darstellung oben oder unten verwiesen.

In einer weiteren, bevorzugten Ausgestaltung kann die Vorrichtung eine an anderer Stelle hierin beschriebenen Bildschirm umfassen, der zur Darstellung des gewünschten Zeichens in der mindestens einen ersten Entfernung und/oder der zweiten Entfernung vor dem mindestens einen Auge eingerichtet ist. Insbesondere um die mindestens eine erste und/oder zweite Entfernung fest, aber wählbar einzustellen, kann der Bildschirm beweglich, jedoch fixierbar angeordnet sein. Alternativ oder zusätzlich kann, wie oben dargestellt, eine Projektionseinrichtung vorgesehen sein, die zur Abbildung des Zeichens auf das mindestens eine Auge des Nutzers eingerichtet ist. Vorzugsweise kann hierbei die Projektionseinrichtung eine Badal-Linse zur Abbildung des Zeichens aufweisen, wobei die Badal-Linse vor dem mindestens einen Auge des Nutzers angeordnet ist. Der Begriff der "Badal-Linse" bezeichnet ein optisches Element, das mindestens eine Linse umfasst und zur Darstellung des Zeichens mit einer stets gleichen Winkelgröße eingerichtet ist.

In einer weiteren, bevorzugten Ausgestaltung kann die Auswerteeinheit eine Einrichtung zur Erfassung eines Abstands des mindestens einen Auges des Nutzers von dem Bildschirm oder der Kamera verfügen. Hierzu kann durch Bildverarbeitung einer Bildfolge, welche von der Kamera insbesondere von der Augenpartie des Nutzers aufgenommen wurde, aus einer Bestimmung einer Pupillendistanz zwischen der Kamera und dem mindestens einen Auge des Nutzers insbesondere dann, wenn eine Kalibrierung der Pixel der Kamera in räumlichen Einheiten vorliegt, eine Ermittlung des Pupillendurchmessers des mindestens einen Auges des Nutzers ausgeführt werden. In einer bevorzugten Ausgestaltung können mindestens zwei Kameras vorgesehen sein, die gemeinsam in Form einer Stereokamera angeordnet sind und daher zur Erfassung des Abstands des mindestens einen Auges des Nutzers von dem Bildschirm eingerichtet sind. Alternativ oder zusätzlich kann die Vorrichtung einen Entfernungsmesser umfassen, der zur Bestimmung der Pupillendistanz zwischen der Kamera und dem mindestens einen Auge des Nutzers eingerichtet ist.

In einer weiteren, bevorzugten Ausgestaltung kann die Vorrichtung zwei gesonderte Einrichtungen zur Darstellung eines Zeichens, zwei gesonderte Augenbewegungsmesseinrichtungen und, optional, zwei gesonderte Refraktionsmesseinrichtungen umfassen, die zur gleichzeitigen, gemeinsamen Bestimmung der Akkommodation und der Vergenz der beiden Augen des Nutzers eingerichtet sein können.

Für Definitionen und optionale Ausgestaltungen der Vorrichtung einschließlich der darin aufgeführten Merkmale wird auf die Beschreibung des Verfahrens zur gemeinsamen Bestimmung der Akkommodation und der Vergenz des mindestens einen Auges des Nutzers in diesem Dokument verwiesen.

In einer bevorzugten Ausführungsform kann die Vorrichtung als wenigstens ein mobiles Endgerät ausgestaltet sein. Für Definitionen und optionale Ausgestaltungen der Vorrichtung als wenigstens ein mobiles Endgerät einschließlich der darin aufgeführten Merkmale wird auf die Beschreibung des Verfahrens zur gemeinsamen Bestimmung der Akkommodation und der Vergenz des mindestens einen Auges des Nutzers in diesem Dokument mittels wenigstens eines mobilen Endgeräts verwiesen.

Während die US 2012/0287398 A1 dazu eingerichtet ist, die Refraktion der Augen eines Nutzers zu bestimmen, wobei optional eine Kontrolle der Vergenz vorgesehen sein kann, ermöglicht die vorliegende Erfindung die gemeinsame Bestimmung der Akkommodation und der Vergenz eines oder beider Augen des Nutzers. Das erfindungsgemäße Verfahren und die vorgeschlagene Vorrichtung können daher, wie unten näher beschrieben, insbesondere zur Myopie-Kontrolle eines oder beider Augen des Nutzers und bei der Herstellung eines Brillenglases oder einer Kontaktlinse für das Auge oder für die Augen des betreffenden Nutzers eingesetzt werden.

Die vorliegende Erfindung eignet sich insbesondere zur Ermittlung von Werten für eine Myopie-Kontrolle mindestens eines Auges eines Nutzers, wobei hierzu das hierin beschriebene Verfahren zur gemeinsamen Bestimmung von Akkommodation und Vergenz des mindestens einen Auges eines Nutzers eingesetzt wird, wobei die gemeinsam bestimmte Akkommodation und Vergenz des mindestens einen Auges des Nutzers als die Werte für die Myopie-Kontrolle verwendet werden. Hierbei kann insbesondere eine optimierte, individualisierte Versorgung von kurzsichtigen Probanden mit individualisierten optischen Lösungen zur Myopie-Kontrolle erfolgen.

In einer bevorzugten Ausgestaltung betrifft dies die Ermittlung von Werten für eine Myopie-Kontrolle der Augen eines Nutzers, insbesondere zur gemeinsamen und besonders bevorzugt gleichzeitigen Bestimmung von Akkommodation und Vergenz der Augen eines Nutzers, wobei die gemeinsam und besonders bevorzugt gleichzeitig bestimmte Akkommodation und Vergenz der Augen des Nutzers als die Werte für die Myopie-Kontrolle verwendet werden.

In einer weiteren bevorzugten Ausgestaltung erfolgt die Ermittlung von Werten für eine Myopie-Kontrolle mindestens eines Auges eines Nutzers mittels wenigstens einen mobilen Endgeräts, wobei hierzu das hierin beschriebene Verfahren mittels wenigstens einen mobilen Endgeräts zur gemeinsamen

Bestimmung von Akkommodation und Vergenz des mindestens einen Auges eines Nutzers eingesetzt wird, wobei die gemeinsam bestimmte Akkommodation und Vergenz des mindestens einen Auges des Nutzers als die Werte für die Myopie-Kontrolle verwendet werden.

In einer bevorzugten Ausgestaltung können das vorgeschlagene Verfahren und die hierin vorgestellte Vorrichtung zunächst als Screening-Tool, auch beispielsweise durch Verwendung wenigstens eines mobilen Endgeräts, eingesetzt werden, um individuelle Voraussetzungen einzelner kurzsichtiger Nutzer und die Erfolgsaussichten hinsichtlich einer Verringerung einer Progression der Myopie mittels zusätzlicher optischer Einrichtungen, insbesondere mittels Gleitsichtgläsern, abzuschätzen. Im Rahme einer Erstversorgung können hierbei Parameter der optischen Einrichtungen, etwa eine Stärke einer Addition und eines Insets im Falle von Gleitsichtgläsern, individuell auf die gemeinsam bestimmte Akkommodation und der Vergenz abstimmt werden.

Im Anschluss an die Erstversorgung können das vorgeschlagene Verfahren und die vorgestellte Vorrichtung zur Verlaufskontrolle verwendet werden. Ändert sich die gemeinsam bestimmte Akkommodation und Vergenz eines Nutzers über die Zeit, so lassen sich die Parameter der optischen Einrichtungen, etwa die Stärke der Addition und/oder des Insets im Falle von Gleitsichtgläsern, individuell an den Verlauf der Progression der Kurzsichtigkeit anpassen.

In einem Fallbeispiel kann angenommen werden, dass ein kurzsichtiger Nutzer mit einer Fehlsichtigkeit von -3,0 D eine Akkommodationsungenauigkeit von 0,75 D aufweist, wobei eine Genauigkeit der Vergenz von 4 ΔD/D als normal betrachtet werden kann. Dieser Nutzer wird mit einem speziell für die Myopie-Kontrolle eingerichtetem Gleitsichtglas versorgt, wobei regelmäßig Kontrollmessungen im Rahmen einer Verlaufskontrolle durchgeführt werden. Die eingestellte Addition des Gleitsichtglases verringert die Ungenauigkeit der Akkommodation auf 0,25 D und kann ein Fortschreiten der Kurzsichtigkeit verlangsamen. Nach einem Zeitraum von z.B. 12 Monaten wird festgestellt, dass der Wert der Ungenauigkeit der Akkommodation wieder dem Wert vor der Erstversorgung entspricht, woraufhin der Wert der Addition individuell angepasst werden kann.

Damit können die vorliegenden Verfahren und Vorrichtungen in besonders bevorzugter Weise auch als Vorhersagewert für eine Entwicklung von Fehlsichtigkeiten bei dem Nutzer verwendet werden.

Die Anwendung des vorgeschlagenen Verfahrens und der vorliegenden Vorrichtung als Screening-Tool und/oder zur Verlaufskontrolle im Rahmen einer Myopie-Kontrolle lässt sich grundsätzlich durch ein Vorhandensein von Normwerten verbessern, mit deren Hilfe ein Anwender in der Lage ist, zu entscheiden, ob und in welcher Form eine Anpassung der Myopie-Kontrolle erforderlich ist. Bis derartige Normwerte zur Verfügung stehen, können das vorgeschlagene Verfahren und die vorliegende Vorrichtung als standardisiertes Untersuchungsverfahren bzw. Untersuchungsgerät dienen.

Wie bereits erwähnt, eignen sich das hier vorgeschlagene Verfahren und die hier vorgestellte Vorrichtung zur gemeinsamen Bestimmung von Akkommodation und Vergenz mindestens eines Auges eines Nutzers insbesondere zur Verwendung in einem Verfahren zur Herstellung eines Brillenglases für das mindestens eine Auge des betreffenden Nutzers. Unter einem "Brillenglas" wird gemäß der Norm, Abschnitte 8.1.1 und 8.1.2 eine optische Linse verstanden, welche zur Korrektion von Fehlsichtigkeiten des Auges dienen soll, wobei die optische Linse vor dem Auge des Nutzers, aber nicht in Kontakt mit dem Auge getragen wird. Gemäß diesem weiteren Aspekt der vorliegenden Erfindung erfolgt die Herstellung des Brillenglases durch Bearbeitung eines Linsenrohlings oder eines Brillenglas-Halbfertigprodukts, wobei der Linsenrohling oder das Brillenglas-Halbfertigprodukt anhand von Refraktionsdaten bearbeitet wird, wobei die Refraktionsdaten Werte für eine Akkommodation und eine Vergenz des mindestens einen Auges des Nutzers berücksichtigen, die gemäß dem hierin beschriebenen Verfahren zur gemeinsamen Bestimmung der Akkommodation und der Vergenz des mindestens einen Auges des Nutzers bestimmt werden. Für weitere Ausgestaltungen des Verfahrens zur Herstellung eines Brillenglases wird auf die oben oder untenstehende Beschreibung des Verfahrens und der Vorrichtung zur gemeinsamen Bestimmung der Akkommodation und der Vergenz des mindestens einen Auges des Nutzers verwiesen. Die Refraktionsdaten umfassen bevorzugt die als Ergebnis der Refraktionsbestimmung spezifizierte dioptrische Wirkung zur Korrektion des Sehvermögens des mindestens einen Auges des Nutzers. Die dioptrische Wirkung ist gemäß der Norm, Abschnitt 9.3, der Sammelbegriff für die fokussierende und die prismatische Wirkung eines Brillenglases.

Die erfindungsgemäßen Verfahren und die vorgeschlagene Vorrichtung weisen gegenüber herkömmlichen Vorrichtungen und Verfahren zahlreiche Vorteile auf. In besonders vorteilhafter Weise kann eine objektive gemeinsame Bestimmung der Akkommodation und der Vergenz des mindestens einen Auges des Nutzers ohne Spezialgeräte erfolgen, insbesondere ohne dass ein subjektives Feedback durch den Nutzer, z.B. in Form einer manuellen oder akustischen Eingabe in die Vorrichtung, erforderlich wäre. Außerdem ist keine Bedienung durch Fachpersonal notwendig. Weiterhin können die vorliegenden Verfahren und die vorgeschlagene Vorrichtung als Screening-Tool zur Untersuchung eines möglichen Risikos im Hinblick auf eine Entwicklung zur Kurzsichtigkeit und/oder zur Verlaufskontrolle einer bereits bestehenden Kurzsichtigkeit, bevorzugt unter dem Einfluss von zusätzlichen optischen Einrichtungen, insbesondere von Gleitsichtgläsern, eingesetzt werden.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigen:
- Figur 1: ein bevorzugtes Ausführungsbeispiel einer erfindungsgemäßen Vorrichtung zur gemeinsamen Bestimmung von Akkommodation und Vergenz mindestens eines Auges eines Nutzers;
- Figur 2: ein besonders bevorzugtes Ausführungsbeispiel der erfindungsgemäßen Vorrichtung zur gemeinsamen Bestimmung von Akkommodation und Vergenz des mindestens einen Auges des Nutzers;
- Figur 3: eine schematische Darstellung der Funktionsweise des erfindungsgemäßen Verfahrens zur gemeinsamen Bestimmung der Akkommodation und der Vergenz des mindestens einen Auges des Nutzers; und
- Figur 4: ein bevorzugtes Ausführungsbeispiel des erfindungsgemäßen Verfahrens zur gemeinsamen Bestimmung der Akkommodation und der Vergenz des mindestens einen Auges des Nutzers.

### Ausführungsbeispiele

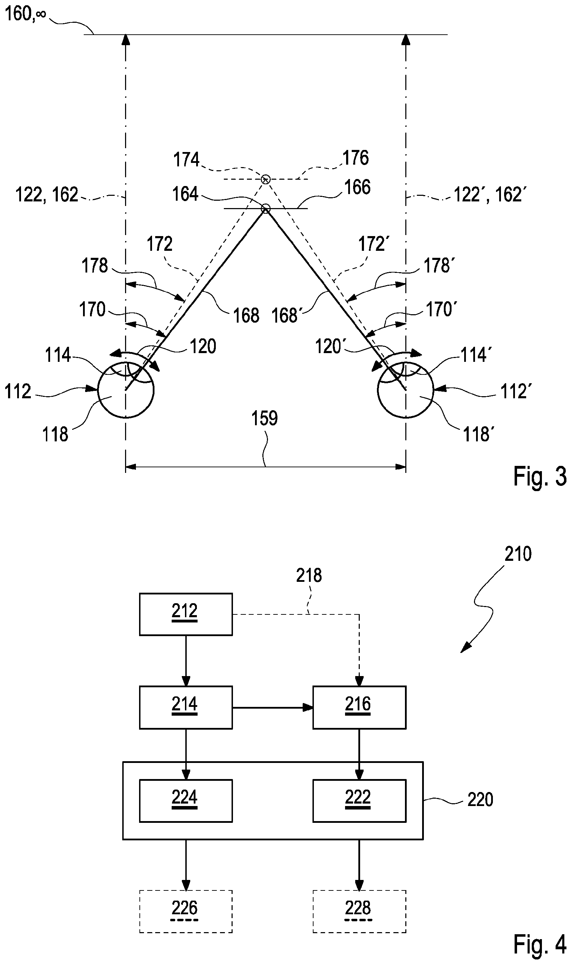

Figur 1 zeigt in schematischer Darstellung ein bevorzugtes Ausführungsbeispiel einer erfindungsgemäßen Vorrichtung 110 zur gemeinsamen Bestimmung von Akkommodation und Vergenz eines oder beider Augen 112, 112' eines Nutzers. Hierbei ist in Figur 1 schematisch nur ein einzelnes Auge 112 des Nutzers dargestellt, wobei das Auge 112 eine Pupille 114 aufweist, durch welche ein Lichtstrahl 116 in einen Innenraum 118 des Auges 112 einfällt. Hierdurch erfolgt eine als "Refraktion" bezeichnete Lichtbrechung im Auge 112 des Nutzers. Weiterhin kann das Auge 112 eine Augenrotation 120 ausführen. Wie in Figur 3 näher dargestellt, werden gegensinnige Augenbewegungen der beiden Augen 112, 112' eines Augenpaars des Nutzers, wobei jedes der beiden Augen 112, 112' eine Augenrotation 120 um zueinander parallele Achsen 122 in eine jeweils entgegengesetzte Rotationsrichtung ausführt, als "Vergenz" bezeichnet.

Die vorgeschlagene Vorrichtung 110 umfasst eine Einrichtung 124 zur Darstellung eines Zeichens in mindestens einer gewünschten Entfernung vor dem Auge 112 des Nutzers, wobei das Zeichen (nicht dargestellt) zu einer Stimulation der Akkommodation des Auges 112 geeignet ist. Das Zeichen umfasst hierbei ein Sehzeichen, ausgewählt aus einem oder mehreren Buchstaben, Zahlen, Symbolen, Bildern oder Mustern, die farbig oder in schwarz-weiß dargestellt werden können. Die Darstellung des Zeichens kann monokular, d.h. jeweils getrennt für ein Auge 112, oder binokular, d.h. gemeinsam und simultan für beide Augen 112, 112' eines Augenpaars, erfolgen. Hierbei kann wie in Figur 1 schematisch dargestellt, die Einrichtung 124 eine optionale feste Linse 126, eine bewegliche Linse 128 und einen Bildschirm 130 aufweisen. Eine andere Ausführung der Einrichtung 124 ist jedoch möglich. In der Ausführung gemäß Figur 1 wird, wie in Figur 1 dargestellt, das Zeichen mittels der Einrichtung 124 derart auf das Auge 112 projiziert, dass der Nutzer das Zeichen virtuell an der vorbestimmten Stelle im Raum, welche der gewünschten Entfernung vor dem Auge 112 des Nutzers entspricht, erkennen kann. In einer alternativen Ausführung (nicht dargestellt) kann die Vorrichtung 110 eine Projektionseinrichtung aufweisen, die dazu eingerichtet ist, das Zeichen an die vorbestimmte Stelle im Raum, die der gewünschten Entfernung vor dem Auge 112 des Nutzers entspricht, zu projizieren. In einer weiteren alternativen Ausführung (nicht dargestellt) kann die Einrichtung 124 einen in der gewünschten Entfernung vor dem Auge 112 des Nutzers angeordneten Bildschirm aufweisen. Der Bildschirm kann hierbei ausgewählt sein aus einem Monitor, einem Screen oder einem Display, wobei der Bildschirm für Aufblick oder, vorzugsweise, für Durchblick eingerichtet sein kann. Weitere Ausführungen der Einrichtung 124 sind jedoch denkbar.

Weiterhin kann die Einrichtung 124 zur Darstellung des Zeichens des Nutzers dazu eingerichtet sein, einen Parameter des Zeichens zu verändern, wobei der Parameter eine Eigenschaft des Zeichens betrifft, je nach Zeichen, ausgewählt aus einer Ausdehnung, Orientierung, Frequenz, Kontrast oder Farbe, einschließlich schwarz und weiß, des Zeichens oder eines Teils davon. Im Falle eines periodischen Musters kann sich der Parameter auf eine wiederholt dargestellte Struktur beziehen, insbesondere auf eine Anordnung von periodischen Maxima oder Minima, insbesondere auf mindestens eine Ortsfrequenz des periodischen Musters. Weiterhin kann die Einrichtung 124 zur Darstellung des Zeichens dazu eingerichtet sein, eine Bewegung des Zeichens auszuführen, insbesondere kontinuierlich oder in Sprüngen, wobei bevorzugt der Parameter des dargestellten Zeichens verändert wird, während das dargestellte Zeichen die Bewegung ausführt. Hierbei ist es unerheblich, wenn die Bewegung des Zeichens nur scheinbar erfolgt.

Die vorgeschlagene Vorrichtung 110 umfasst ferner eine Augenbewegungsmesseinrichtung 132, die zur Erfassung einer Augenbewegung des Auges 112 eingerichtet ist und die daher auch als "Eyetracker" bezeichnet wird. Die Augenbewegungsmesseinrichtung 132 kann vorzugsweise eine Kamera 134, besonders bevorzugt eine Videokamera, umfassen, insbesondere um ein videobasiertes "Eyetracking" durchführen zu können, indem Bildfolgen einer Augenpartie des Nutzers aufgenommen und mittels Bildverarbeitung ausgewertet werden, um hieraus mindestens eine Augenbewegungsmetrik zu ermitteln. Hierfür lassen sich insbesondere jeweils bekannte Algorithmen einsetzen. Die Augenbewegungsmetrik bezieht sich hierbei auf ein Maß, das mit der Bewegung des Auges 112 des Nutzers verknüpft ist, wobei die Bewegung des Auges 112 des Nutzers durch das Zeichen, das als Stimulus wirkt, hervorgerufen wird. Die Augenbewegungsmetrik kann ausgewählt sein aus: gegensinnigen Augenbewegungen der beiden Augen 112, 112' des Nutzers; einer Augenfolgebewegung; einer Augenbewegung, die sich auf Mikrosakkaden bezieht, die eine Mikrosakkadenrichtung, eine Mikrosakkadenrate oder eine Sakkadengenauigkeit umfasst; oder einem optokinetischen Nystagmus. Weitere Arten von Augenbewegungsmetriken, zum Beispiel eine Fixationsdauer, sind jedoch möglich. Eine Auswahl der Augenbewegungsmetriken hängt im Wesentlichen ab von einer Genauigkeit der Augenbewegungsmesseinrichtung 132 sowie von dem jeweiligen Verwendungszweck. Während sich gegensinnige Augenbewegungen insbesondere zur Bestimmung der Vergenz verwenden lassen, können sich Augenfolgebewegungen bevorzugt zur Bestimmung der Refraktion eignen.

Aus den aufgenommenen Bildfolgen können mittels Bildverarbeitung zudem geometrische Daten des Auges 112, bevorzugt der Pupille 114, insbesondere Position und Durchmesser der Pupille 114, ermittelt werden und hieraus zum Beispiel die Blickrichtung des Auges 112 bestimmt werden. Hierzu sind Verfahren einsetzbar, welche ausgewählte Reflexionspunkte einbeziehen, die auf Vorderseite und/oder Rückseite von Cornea und Linse entstehen können, wenn das Auge 112 durch eine Lichtquelle angestrahlt wird. Insbesondere kann ein Hornhautreflex oder ein anderer Reflex aufgenommen werden. Da sich der Hornhautreflex ohne Kopfbewegung nicht bewegt, jedoch die Pupille 114 ihre Position bei der Augenbewegung ändert, kann hieraus auf die Augenrotation 120 geschlossen werden. Erfindungsgemäß dient das Erfassen der Augenbewegung, insbesondere der Augenrotation 120, dazu, die Vergenz zu bestimmen, indem die gegensinnigen Augenbewegungen der beiden Augen 112, 112' des Nutzers, wobei jedes der beiden Augen 112, 112' eine Augenrotation 120 um zueinander parallele Achsen 122, 122' in eine entgegengesetzte Rotationsrichtung ausführt, erfasst und ausgewertet werden.

Wie in der europäischen Patentanmeldung Az. 19 170 561.5, angemeldet am 23.04.2019, offenbart, kann das Erfassen der Augenbewegung des Auges 112 des Nutzers in Abhängigkeit von dem Zeichen erfolgen, während mindestens ein Parameter des Zeichens verändert wird. Hierzu können Daten zur Erfassung der Augenbewegung des Nutzers, welche die Kamera 134 aufgenommen hat, dazu verwendet werden, um die Blickrichtung des Nutzers auf das dargestellte Zeichen zu ermitteln. Im Falle einer zunehmenden Abnahme eines der Parameter des Zeichens, insbesondere der Ortsfrequenz in dem periodischen Muster, wird die Augenfolgebewegung des Nutzers solange der Bewegung des Zeichens entsprechen, solange der Nutzer das dargestellte Zeichen erkennen kann. Ist ein Zeitpunkt erreicht, zu dem der Nutzer das dargestellte Zeichen, insbesondere das periodische Muster, gerade nicht mehr erkennen kann und das somit nicht mehr als Stimulus für das Auge 112 des Nutzers wirken kann, weicht die Augenfolgebewegung des Nutzers von der Bewegung des Zeichens ab. Wird umgekehrt der Zeitpunkt erreicht, zu dem der Nutzer das dargestellte Zeichen, insbesondere das periodische Muster, erstmals gerade erkennen kann und das somit erstmals als Stimulus für das Auge 112 des Nutzers wirkt, beginnt die Augenfolgebewegung des Nutzers damit, der Bewegung des Zeichens zu folgen. Unabhängig von der Art der Ausgestaltung kann hierbei bevorzugt eine Schwelle festgesetzt werden, mittels der ein Grad der Abweichung der Augenfolgebewegung des Nutzers von der Bewegung des Zeichens als der gesuchte Zeitpunkt festgelegt wird. Der Zeitpunkt, zu dem die Abweichung die festgelegte Schwelle überschreitet oder unterschreitet, stellt hierbei den gesuchten Zeitpunkt dar. Aus der Feststellung des Zeitpunkts kann nunmehr die Bestimmung eines Wertes für die Refraktion des Auges 112 des Nutzers aus dem Wert des Parameters erfolgen, der zu dem festgestellten Zeitpunkt dazu verwendet wurde, den Parameter des Zeichens einzustellen. Alternativ kann hierzu die Angabe eines Refraktionsvektors erfolgen.

Die vorgeschlagene Vorrichtung 110 umfasst weiterhin eine Auswerteeinheit 136, die zur gemeinsamen Bestimmung der Akkommodation aus der Änderung der Refraktion und der Vergenz aus der Augenbewegung eingerichtet ist. Hierbei kann eine drahtgebundene oder eine drahtlose Verbindung 138 zwischen der Augenbewegungsmesseinrichtung 132 und der Auswerteeinheit 136 vorgesehen sein. Eine weitere Verbindung 138 kann zwischen der Auswerteeinheit 136 und der Einrichtung 124 zur Darstellung des Zeichens bestehen. Auf diese Weise kann die Auswerteeinheit 136 auch zur Ansteuerung der Augenbewegungsmesseinrichtung 132 und der Einrichtung 124 zur Darstellung des Zeichens eingesetzt werden, insbesondere um den Parameter des Zeichens einzustellen. Weiterhin können Ergebnisse aus der gemeinsamen Bestimmung der Akkommodation und der Vergenz dem Nutzer oder einer anderen Person, insbesondere einem Augenoptiker oder Augenarzt, zur Verfügung gestellt werden, beispielsweise mittels eines Monitors 140. Weiterhin kann eine Tastatur 142 zur Eingabe von Werten für die oben genannte Ansteuerung vorgesehen sein. Weitere Arten der Ausführung der Auswerteeinheit 136 sind jedoch möglich.

Erfindungsgemäß ist die Auswerteeinheit 136 dazu eingerichtet, die gemeinsame Bestimmung der Akkommodation aus der Änderung der Refraktion und der Vergenz aus der Augenbewegung auszuführen. Hierzu werden Daten aus der Erfassung der Augenbewegung des Nutzers, welche die Augenbewegungsmesseinrichtung 132 aufgenommen hat, an die Auswerteeinheit 136 weitergeleitet. Weiterhin sind aufgrund der elektronischen Ansteuerung der Einrichtung 124 die Parameter des Zeichens 122 bekannt und können somit von der Auswerteeinheit 136 zur gewünschten Auswertung verwendet werden. Weitere Details zur Bestimmung der Akkommodation und der Vergenz finden sich unten in der Beschreibung zu Figur 3.

Figur 2 zeigt in schematischer Darstellung ein besonders bevorzugtes Ausführungsbeispiel der erfindungsgemäßen Vorrichtung 110 zur gemeinsamen Bestimmung der Akkommodation und der Vergenz von Augen 112, 112' eines Nutzers. Wie in Figur 2 dargestellt, kann die Refraktion des Auges besonders bevorzugt mittels einer Refraktionsmesseinrichtung 144, die zur Erfassung der Refraktion des Auges 112 eingerichtet ist, ermittelt werden, wobei die Ansteuerung der Refraktionsmesseinrichtung 144 insbesondere unter Verwendung der Auswerteeinheit 136 erfolgen kann. Die Refraktionsmesseinrichtung 144 kann hierbei eine Linse 146, eine Blende 148 und einen optischen Sensor 150 umfassen, die zur Bestimmung der Defokussierung des Auges 112 des Nutzers eingerichtet sind. Andere Arten der Ausgestaltung der Refraktionsmesseinrichtung 144 sind jedoch möglich. Zur Beaufschlagung der Refraktionsmesseinrichtung 144 mit einem Teil des Lichtstrahls 152, der von dem Auge 112 reflektiert wird, können, wie Figur 2 schematisch zeigt, zusätzlich ein Strahlteiler 154, insbesondere ein teiltransparenter Spiegel 156, und ein intransparenter Umlenkspiegel 158 vorgesehen sein. Andere oder weitere optische Elemente für diesen Zweck sind jedoch denkbar. Für weitere Einzelheiten der in Figur 2 dargestellten Vorrichtung 110 wird auf die obige Beschreibung der Vorrichtung 110 gemäß Figur 1 verwiesen.

In einer weiteren, bevorzugten Ausgestaltung kann die Vorrichtung 110 nach den Figuren 1 oder 2 zwei gesonderte Einrichtungen 124 zur Darstellung eines Zeichens, zwei gesonderte Augenbewegungsmesseinrichtungen 132 und, darüber hinaus in Figur 2, zwei gesonderte Refraktionsmesseinrichtungen 144 umfassen, die zur gleichzeitigen, gemeinsamen Bestimmung der Akkommodation der Vergenz der beiden Augen 112, 112' des Nutzers eingerichtet sein können.

Figur 3 zeigt eine schematische Darstellung der Funktionsweise des erfindungsgemäßen Verfahrens zur gemeinsamen Bestimmung der Akkommodation und der Vergenz der Augen 112, 112' des Nutzers, die sich in einer Pupillendistanz 159 (abgekürzt "IPD" von engl. *interpupillary distance*) befinden und jeweils eine Pupille 114, 114' aufweisen. In dieser Darstellung fixieren die beiden Augen 112, 112' des Nutzers zunächst jeweils ein Ziel in einer zweiten Entfernung 160, das hier im Unendlichen ∞ liegt. Hierbei sind die Blickrichtungen 162, 162' der beiden Augen 112, 112' jeweils entlang der zueinander parallelen Achsen 122, 122' ausgerichtet. Ein anderer Wert für die zweite Entfernung 160 ist jedoch möglich, zum Beispiel ein Wert von mindestens 1 m, bevorzugt mindestens 1,5 m, besonders bevorzugt mindestens 2 m, bei welchen das Auge 112 im Wesentlichen einen akkommodationslosen Zustand annimmt.

In der weiteren Darstellung gemäß Figur 3 sollen die beiden Augen 112, 112' des Nutzers anschließend ein Ziel 164 in einer ersten Entfernung 166 fixieren, um das sich am Ziel 164 befindliche Zeichen möglichst scharf auf die Netzhautebene des Auges abbilden zu können. Bevorzugt können hierzu mindestens zwei, bevorzugt mindestens drei, mindestens vier, mindestens fünf oder mindestens sechs Werte für die erste Entfernung 166 verwendet werden, wobei bevorzugt Werte von 15 cm bis 60 cm, besonders bevorzugt von 20 cm bis 50 cm, insbesondere bei etwa 20 cm, 25 cm, 40 cm und 50 cm, ausgewählt werden, bei welchen ein akkommodierter Zustand des Auges vorliegt. Für das hier beispielhaft vorgestellte Ausführungsbeispiel wird ein Wert von 40 cm für die erste Entfernung 160 angesetzt; andere Werte sind jedoch möglich. Durch die Wahl von zwei oder mehr voneinander verschiedenen ersten Entfernungen 166 kann in vorteilhafter Weise eine Messkurve erhalten werden, aus welcher eine Auswertung mit erhöhter Genauigkeit, insbesondere aus einer Ermittlung einer Steigung der Messkurve, ermöglicht wird.

Für den Fall, dass die beiden Augen 112, 112' keinen Refraktionsfehler aufweisen, würden die beiden Augen 112, 112', wie Figur 3 schematisch zeigt, geänderte Blickrichtungen 168, 168' (durchgezogene Linien) annehmen, um das Ziel 164 in der ersten Entfernung 166 zu fixieren. Bei dem hier beispielhaft angesetzten Wert von 40 cm für die erste Entfernung 160 entspricht dies einer Akkommodation von 2,5 D. Eine derartige Änderung der Blickrichtungen 162, 162' zu den geänderten Blickrichtungen 168, 168' würde einem Konvergenzbedarf 170, 170' der beiden Augen 112, 112' um den in Figur 3 angegebenen Winkel entsprechen, was als "Vergenz" bezeichnet wird. Hierzu müssten die beiden Augen 112, 112' die gegensinnigen Augenrotationen 120, 120' in eine jeweils entgegengesetzte Rotationsrichtung um den angegebenen Winkel um die zueinander parallelen Achsen 122, 122' ausführen.

Wie oben bereits erwähnt, kann zur Ermittlung der Vergenz jeweils ein Hornhautreflex der Pupillen 114, 114' der beiden Auge 112, 112' aufgenommen werden. Dies ist insbesondere daher von Vorteil, weil sich der jeweilige Hornhautreflex ohne Kopfbewegung nicht bewegt, jedoch die Pupillen 114, 114' ihre jeweilige Position bei der Augenbewegung so ändern, dass hieraus zuverlässig auf die zugehörige Augenrotation 120, 120' geschlossen werden kann. Andere Arten der Ermittlung der Vergenz der beiden Augen 112, 112' sind jedoch möglich.

Weisen die beiden Augen 112, 112' jedoch jeweils einen Refraktionsfehler auf, würden die beiden Augen 112, 112', wie in Figur 3 schematisch dargestellt, jeweils abweichend geänderte Blickrichtungen 172, 172' (gestrichelte Linien) annehmen. Würde beispielsweise die Akkommodation nur 2,3 D anstatt 2,5 D betragen, so läge hier ein Akkommodationsfehler von 0,2 D vor. Hierdurch würde sich das sich in der ersten Entfernung 166 befindliche Ziel 164 scheinbar an einem anderen Ort 174 in einer scheinbaren ersten Entfernung 176 befinden. Die dadurch bewirkte Änderung der Blickrichtungen 162, 162' zu den abweichend geänderten Blickrichtungen 172, 172' würde einem abweichend Konvergenzbedarf 178, 178' der beiden Augen 112, 112' um den in Figur 3 ebenfalls angegebenen Winkel entsprechen.

Um jedoch das Ziel 164 in der richtigen ersten Entfernung 166 fixieren zu können, benötigt der Nutzer somit jeweils eine Korrektur der Refraktion der beiden Augen 112, 112' um einen Wert von 0,2 D unter Beachtung der entsprechenden Vergenz der beiden Augen 112, 112' bei der beabsichtigten Fixierung der beiden Augen 112, 112' auf das Ziel 164 in der ersten Entfernung 166. Damit kann die hier gewünschte gemeinsame Bestimmung 220 der Akkommodation und der Vergenz der Augen 112, 112' durch das Ermitteln der Änderung der Refraktion der Augen 112,112' bei der Akkommodation in die erste Entfernung 166 gegenüber der Akkommodation in die zweite Entfernung 160 erfolgen, was hier beispielhaft einem Wert von 0,2 D entspricht. Damit lässt sich insbesondere ein Wert für den Betrag der Vergenz AC, der für eine bestimmte Akkommodationsentfernung A eingestellt wird (AC/A), bestimmen.

Figur 4 zeigt schematisch ein Ablaufdiagramm eines bevorzugten Ausführungsbeispiels eines erfindungsgemäßen Verfahrens 210 zur gemeinsamen Bestimmung der Akkommodation und der Vergenz der Augen des Nutzers.

Gemäß Schritt a) erfolgt hierbei zunächst eine Darstellung 212 des Zeichens in mindestens einer ersten Entfernung vor dem Auge 112 des Nutzers zur Stimulation der Akkommodation des Auges 112. Hierzu kann insbesondere die Einrichtung 124 zur Darstellung eines Zeichens, das zu einer Stimulation der Akkommodation des Auges 112 geeignet ist, in der mindestens einen gewünschten Entfernung vor dem Auge 112 des Nutzers eingesetzt werden.

Gemäß Schritt b) erfolgt, vorzugsweise hieran anschließend, eine Erfassung 214 der Augenbewegung des Auges 112 des Nutzers in Abhängigkeit von dem dargestellten Zeichen. Hierzu kann insbesondere die Augenbewegungsmesseinrichtung 132, die zur Erfassung einer Augenbewegung des Auges 112 eingerichtet ist und die daher vorzugsweise eine Kamera 134, besonders bevorzugt eine Videokamera, umfassen kann, eingesetzt werden. Erfindungsgemäß dient das Erfassen einer Augenbewegung dazu, die gesuchte Vergenz zu bestimmen, indem gegensinnige Augenbewegungen der beiden Augen 112 eines Augenpaars des Nutzers, wobei jedes der beiden Augen 112 eine Augenrotation 120 um zueinander parallele Achsen 122 in eine jeweils entgegengesetzte Rotationsrichtung ausführt, erfasst und ausgewertet werden.

Gemäß Schritt c) erfolgt eine Ermittlung 216 der Refraktion des Auges 112 bei der Akkommodation des Auges 112 in die mindestens eine erste Entfernung. In einer bevorzugten Ausgestaltung der vorliegenden Erfindung kann die erfolgte Erfassung 214 der Augenbewegung des Auges 112 des Nutzers zusätzlich auch zur Ermittlung 216 der Refraktion des Auges 112 verwendet werden, wozu beispielsweise das in der europäischen Patentanmeldung Az. 19 170 561.5, angemeldet am 23.04.2019, offenbarte Verfahren eingesetzt werden kann. Alternativ oder zusätzlich kann eine Messung 218 der Refraktion des Auges 112 mittels der oben beschriebenen Refraktionsmesseinrichtung 144, die zur Erfassung der Refraktion des Auges 112 bei der Akkommodation des Auges 112 auf das auf das Auge 112 als Stimulus wirkende Zeichen eingerichtet ist, vorgenommen werden.

Schließlich erfolgt gemäß Schritt d) die gemeinsame Bestimmung 220 der Akkommodation und der Vergenz des Auges 112 des Nutzers durch ein Ermitteln 222 einer Änderung der Refraktion des Auges 112 bei der Akkommodation des Auges 112 in die mindestens eine erste Entfernung gegenüber der Akkommodation des Auges 112 in eine zweite Entfernung und durch Ermitteln 224 der Vergenz des Auges 112 aus der Augenbewegung des Auges 112, insbesondere bei der Augenrotation 120 jedes der beiden Augen 112 um zueinander parallele Achsen 122 in eine jeweils entgegengesetzte Rotationsrichtung, bei der Akkommodation des Auges 112 in die mindestens eine erste Entfernung.

In einer besonderen Ausführung, die bevorzugt bei einer Verlaufskontrolle der beiden Augen 112, 112' des Nutzers über einen Zeitraum, beispielsweise über ein oder mehrere Wochen, Monate, Quartale oder Jahre, zum Einsatz kommen kann, kann auf eine aktuelle Ermittlung 218, insbesondere eine aktuelle Messung 218, der Refraktion bei der Akkommodation der Augen 112, 112' in die zweite Entfernung 160 verzichtet werden und stattdessen ein bekannter Wert für die Refraktion bei der Akkommodation in die zweite Entfernung 160, bevorzugt aus einer früheren Ermittlung 216 dieses Wertes, verwendet werden.

Die mittels des Verfahrens 210 gewonnenen Werte für die Akkommodation und die Vergenz der Augen 112, 112' des Nutzers können vorzugsweise als Vorhersagewert 226 für eine Entwicklung von Fehlsichtigkeiten bei dem Nutzer verwendet werden.

Ebenso können diese Werte bei einer Ermittlung von Refraktionsdaten 228 berücksichtigt werden, die dazu verwendet werden, um einen Linsenrohling oder ein Brillenglas-Halbfertigprodukt in einem Verfahren zur Herstellung von Brillengläsern für die Augen 112, 112' des Nutzers zu bearbeiten. Hierbei kann aus der Bestimmung der Refraktion und der Vergenz insbesondere eine sphärozylindrische Linse ermittelt werden, die als Brillenglas dazu benutzt werden kann, um Refraktionsfehler, die in Folge der Defokussierung der Augen 112, 112' auftreten, so zu kompensieren, dass sich eine möglichst optimale Bildqualität für den Nutzer erzielen lässt.

### Bezugszeichenliste

- 110: Vorrichtung
- 112,112': Auge
- 114, 114': Pupille
- 116: Lichtstrahl
- 118: Innenraum
- 120, 120': Augenrotation
- 122, 122': Achse
- 124: Einrichtung zur Darstellung eines Zeichens
- 126: feste Linse
- 128: bewegliche Linse
- 130: Bildschirm
- 132: Augenbewegungsmesseinrichtung (Eyetracker)
- 134: (Video-)Kamera
- 136: Auswerteeinheit
- 138: Verbindung
- 140: Monitor
- 142: Tastatur
- 144: Refraktionsmesseinrichtung
- 146: Linse
- 148: Blende
- 150: optischer Sensor
- 152: reflektierter Lichtstrahl
- 154: Strahlteiler
- 156: teiltransparenter Spiegel
- 158: Umlenkspiegel
- 159: Pupillendistanz
- 160: zweite Entfernung
- 162, 162': Blickrichtung
- 164: Ziel
- 166: erste Entfernung
- 168, 168': geänderte Blickrichtung
- 170, 170': Konvergenzbedarf
- 172, 172': abweichend geänderte Blickrichtung
- 174: anderer Ort
- 176: scheinbare erste Entfernung
- 178, 178': abweichender Konvergenzbedarf
- 210: Verfahren
- 212: Darstellung des Zeichens
- 214: Erfassung der Augenbewegung
- 216: Ermittlung der Refraktion
- 218: Messung der Refraktion
- 220: gemeinsame Bestimmung der Akkommodation und der Vergenz
- 222: Ermitteln einer Änderung der Refraktion
- 224: Ermitteln der Vergenz
- 226: Vorhersagewert
- 228: Refraktionsdaten

## Patentansprüche

1. Verfahren (210) zur gemeinsamen Bestimmung von Akkommodation und Vergenz mindestens eines Auges (112, 112') eines Nutzers, wobei das Verfahren (210) die folgenden Schritte umfasst:
a) Darstellen mindestens eines Zeichens in mindestens einer ersten Entfernung (166) vor mindestens einem Auge (112, 112') eines Nutzers zur Stimulation der Akkommodation des mindestens einen Auges (112, 112');
b) Erfassen mindestens einer Augenbewegung des mindestens einen Auges (112, 112'); und
c) Ermitteln einer Refraktion des mindestens einen Auges (112, 112') bei der Akkommodation des mindestens einen Auges (112, 112') in die mindestens eine erste Entfernung (166);
d) gemeinsames Bestimmen der Akkommodation und der Vergenz des mindestens einen Auges (112, 112') durch
o Ermitteln einer Änderung der Refraktion des mindestens einen Auges (112, 112') bei der Akkommodation des mindestens einen Auges (112, 112') in die mindestens eine erste Entfernung (166) gegenüber der Akkommodation des mindestens einen Auges (112, 112') in mindestens eine zweite Entfernung (160); und
o Ermitteln der Vergenz des mindestens einen Auges (112, 112') aus der mindestens einen Augenbewegung des mindestens einen Auges (112, 112') bei der Akkommodation des mindestens einen Auges (112, 112') in die mindestens eine erste Entfernung (166),
**dadurch gekennzeichnet,**
**dass** das Ermitteln der Refraktion des mindestens einen Auges (112, 112') durch Erfassen der mindestens einen Augenbewegung des mindestens einen Auges (112, 112') in Abhängigkeit von dem mindestens einen Zeichen erfolgt, während ein Parameter des mindestens einen Zeichens verändert wird, wobei ein Zeitpunkt festgestellt wird, zu dem sich aus der mindestens einen Augenbewegung eine Erkennungsschwelle des Nutzers für das mindestens eine Zeichen ergibt, wobei die Refraktion des mindestens einen Auges (112, 112') aus dem zu dem Zeitpunkt festgelegten Parameter für das mindestens eine Zeichen ermittelt wird.

2. Verfahren (210) nach Anspruch 1, **dadurch gekennzeichnet, dass** das gemeinsames Bestimmen der Akkommodation und der Vergenz für beide Augen (112, 112') des Nutzers erfolgt.

3. Verfahren (210) nach einem der Ansprüche 1 oder 2, **dadurch gekennzeichnet, dass** ein neu ermittelter Wert für die Refraktion des mindestens einen Auges (112, 112') bei der Akkommodation des mindestens einen Auges (112, 112') in mindestens die zweite Entfernung (160) ermittelt wird oder dass ein bekannter Wert für die Refraktion des mindestens einen Auges (112, 112') bei der Akkommodation des mindestens einen Auges (112, 112') in mindestens die zweite Entfernung (160) verwendet wird.

4. Verfahren (210) nach einem der Ansprüche 1 bis 3, **dadurch gekennzeichnet, dass** die mindestens eine erste Entfernung (166) für einen akkommodierten Zustand des mindestens einen Auges (112, 112') und die mindestens zweite Entfernung (160) für einen akkommodationslosen Zustand des mindestens einen Auges (112, 112') ausgewählt wird, oder dass die mindestens eine erste Entfernung (166) für einen akkommodationslosen Zustand des mindestens einen Auges (112, 112') und die mindestens zweite Entfernung (160) für einen akkommodierten Zustand des mindestens einen Auges (112, 112') ausgewählt wird.

5. Verfahren (210) nach einem der Ansprüche 1 bis 4, **dadurch gekennzeichnet, dass** zum Ermitteln der Vergenz des mindestens einen Auges (112, 112') eine Erfassung einer Augenrotation (120, 120') des mindestens einen Auges (112, 112') während der Akkommodation des mindestens einen Auges (112, 112') in die mindestens eine erste Entfernung (166) erfolgt.

6. Verfahren (210) nach einem der Ansprüche 1 bis 5, **dadurch gekennzeichnet, dass** das Ermitteln der Refraktion des mindestens einen Auges (112, 112') mittels einer Refraktionsmesseinrichtung (144), die zur Erfassung der Refraktion des mindestens einen Auges (112, 112') eingerichtet ist, erfolgt.

7. Verfahren (210) nach einem der Ansprüche 1 bis 6, **dadurch gekennzeichnet, dass** die Augenbewegung des mindestens einen Auges (112, 112') eine Augenbewegungsmetrik aufweist, wobei die Augenbewegungsmetrik ausgewählt wird aus der Gruppe umfassend eine Augenfolgebewegung; eine Augenbewegung, die sich auf Mikrosakkaden bezieht; und einen optokinetischen Nystagmus.

8. Verfahren (210) nach einem der Ansprüche 1 bis 7, **dadurch gekennzeichnet, dass** das Darstellen des mindestens einen Zeichens dadurch erfolgt,
- dass das mindestens eine Zeichen in der ersten Entfernung (166) vor dem mindestens einen Auge (112, 112') angeordnet wird;
- dass das mindestens eine Zeichen auf die erste Entfernung (166) vor dem mindestens einen Auge (112, 112') projiziert wird; und/oder
- dass das mindestens eine Zeichen derart auf das mindestens eine Auge (112, 112') projiziert wird, dass der Nutzer das mindestens eine Zeichen virtuell an der ersten Entfernung (166) vor dem mindestens einen Auge (112, 112') erkennen kann.

9. Computerprogramm, umfassend ausführbare Anweisungen zur Durchführung eines Verfahrens (210) zur gemeinsamen Bestimmung von Akkommodation und Vergenz mindestens eines Auges (112, 112') eines Nutzers, wobei das (210) Verfahren die folgenden Schritte umfasst:
a) Darstellen mindestens eines Zeichens in mindestens einer ersten Entfernung (166) vor mindestens einem Auge (112, 112') eines Nutzers zur Stimulation der Akkommodation des mindestens einen Auges (112, 112') mittels einer Einrichtung (124), die zur Darstellung des mindestens einen Zeichens in mindestens der ersten Entfernung (166) vor mindestens dem einen Auge (112, 112') des Nutzers zur Stimulation der Akkommodation des mindestens einen Auges (112, 112') eingerichtet ist;
b) Erfassen mindestens einer Augenbewegung des mindestens einen Auges (112, 112') mittels einer Augenbewegungsmesseinrichtung (132); und
c) Ermitteln einer Refraktion des mindestens einen Auges (112, 112') bei der Akkommodation des mindestens Auges (112, 112') in die mindestens eine erste Entfernung (166) mittels einer Refraktionsmesseinrichtung (144) und/oder der Augenbewegungsmesseinrichtung (132);
d) gemeinsames Bestimmen der Akkommodation und der Vergenz des mindestens einen Auges (112, 112') mittels einer Auswerteeinheit (136) durch
o Ermitteln einer Änderung der Refraktion des mindestens einen Auges (112, 112') bei der Akkommodation des mindestens einen Auges (112, 112') in die mindestens eine erste Entfernung (166) gegenüber der Akkommodation des mindestens einen Auges (112, 112') in mindestens eine zweite Entfernung (160); und
o Ermitteln der Vergenz des mindestens einen Auges (112, 112') aus der mindestens einen Augenbewegung des mindestens einen Auges (112, 112') bei der Akkommodation des mindestens einen Auges (112, 112') in die mindestens eine erste Entfernung (166),
**dadurch gekennzeichnet,**
**dass** das Ermitteln der Refraktion des mindestens einen Auges (112, 112') durch Erfassen der mindestens einen Augenbewegung des mindestens einen Auges (112, 112') in Abhängigkeit von dem mindestens einen Zeichen erfolgt, während ein Parameter des mindestens einen Zeichens verändert wird, wobei ein Zeitpunkt festgestellt wird, zu dem sich aus der mindestens einen Augenbewegung eine Erkennungsschwelle des Nutzers für das mindestens eine Zeichen ergibt, wobei die Refraktion des mindestens einen Auges (112, 112') aus dem zu dem Zeitpunkt festgelegten Parameter für das mindestens eine Zeichen ermittelt wird.

10. Computerlesbarer Datenträger, auf dem das Computerprogramm nach Anspruch 9 gespeichert ist.

11. Datenträgersignal, welches das Computerprogramm nach Anspruch 9 überträgt.

12. Computerlesbares Medium, umfassend Befehle, die bei der Ausführung durch einen Computer diesen veranlassen, die Schritte eines computerimplementierten Verfahrens zur Herstellung eines Brillenglases für mindestens ein Auge (112, 112') eines Nutzers auszuführen, wobei die Herstellung des Brillenglases durch Bearbeitung eines Linsenrohlings oder eines Brillenglas-Halbfertigprodukts erfolgt, wobei der Linsenrohling oder das Brillenglas-Halbfertigprodukt anhand von Refraktionsdaten (228) bearbeitet wird, wobei die Refraktionsdaten (228) Werte für eine Akkommodation und eine Vergenz mindestens eines Auges (112, 112') des Nutzers berücksichtigen, wobei ein Verfahren (210) zur gemeinsamen Bestimmung der Akkommodation und der Vergenz des Auges (112, 112') des Nutzers eingesetzt wird, das die folgenden Schritte umfasst:
a) Darstellen mindestens eines Zeichens in mindestens einer ersten Entfernung (166) vor mindestens einem Auge (112, 112') eines Nutzers zur Stimulation der Akkommodation des mindestens einen Auges (112, 112');
b) Erfassen mindestens einer Augenbewegung des mindestens einen Auges (112, 112'); und
c) Ermitteln einer Refraktion des mindestens einen Auges (112, 112') bei der Akkommodation des mindestens einen Auges (112, 112') in die mindestens eine erste Entfernung (166);
d) gemeinsames Bestimmen der Akkommodation und der Vergenz des mindestens einen Auges (112, 112') durch
o Ermitteln einer Änderung der Refraktion des mindestens einen Auges (112, 112') bei der Akkommodation des mindestens einen Auges (112, 112') in die mindestens eine erste Entfernung (166) gegenüber der Akkommodation des mindestens einen Auges (112, 112') in mindestens eine zweite Entfernung (160); und
o Ermitteln der Vergenz des mindestens einen Auges (112, 112') aus der mindestens einen Augenbewegung des mindestens einen Auges (112, 112') bei der Akkommodation des mindestens einen Auges (112, 112') in die mindestens eine erste Entfernung (166),
**dadurch gekennzeichnet,**
**dass** das Ermitteln der Refraktion des mindestens einen Auges (112, 112') durch Erfassen der mindestens einen Augenbewegung des mindestens einen Auges (112, 112') in Abhängigkeit von dem mindestens einen Zeichen erfolgt, während ein Parameter des mindestens einen Zeichens verändert wird, wobei ein Zeitpunkt festgestellt wird, zu dem sich aus der mindestens einen Augenbewegung eine Erkennungsschwelle des Nutzers für das mindestens eine Zeichen ergibt, wobei die Refraktion des mindestens einen Auges (112, 112') aus dem zu dem Zeitpunkt festgelegten Parameter für das mindestens eine Zeichen ermittelt wird.

13. Vorrichtung (110) zur gemeinsamen Bestimmung von Akkommodation und Vergenz mindestens eines Auges (112, 112') eines Nutzers, wobei die Vorrichtung (110) umfasst:
- eine Einrichtung (124), die zur Darstellung mindestens eines Zeichens in mindestens einer ersten Entfernung (166) vor mindestens einem Auge (112, 112') eines Nutzers zur Stimulation der Akkommodation des mindestens einen Auges (112, 112') eingerichtet ist;
- eine Augenbewegungsmesseinrichtung (132), die zur Erfassung mindestens einer Augenbewegung des mindestens einen Auges (112, 112') eingerichtet ist; und
- eine Auswerteeinheit (136), die eingerichtet ist zur gemeinsamen Bestimmung der Akkommodation aus der Änderung der Refraktion und der Vergenz aus der Augenbewegung durch
o Ermitteln einer Änderung der Refraktion des mindestens einen Auges (112, 112') bei der Akkommodation des mindestens einen Auges (112, 112') in die mindestens eine erste Entfernung (166) gegenüber der Akkommodation des mindestens einen Auges (112, 112') in mindestens eine zweite Entfernung (160); und
o Ermitteln der Vergenz des mindestens einen Auges (112, 112') aus der mindestens einen Augenbewegung des mindestens einen Auges (112, 112') bei der Akkommodation des mindestens einen Auges (112, 112') in die mindestens eine erste Entfernung (166),
**dadurch gekennzeichnet,**
**dass** die Auswerteeinheit (136) ferner dazu eingerichtet ist, das Ermitteln der Refraktion des mindestens einen Auges (112, 112') durch Erfassen der mindestens einen Augenbewegung des mindestens einen Auges (112, 112') in Abhängigkeit von dem mindestens einen Zeichen durchzuführen, während ein Parameter des mindestens einen Zeichens verändert wird, wobei ein Zeitpunkt festgestellt wird, zu dem sich aus der mindestens einen Augenbewegung eine Erkennungsschwelle des Nutzers für das mindestens eine Zeichen ergibt, wobei die Refraktion des mindestens einen Auges (112, 112') aus dem zu dem Zeitpunkt festgelegten Parameter für das mindestens eine Zeichen ermittelt wird.

14. Vorrichtung (110) nach Anspruch 13, **dadurch gekennzeichnet, dass** die die Augenbewegungsmesseinrichtung (132) eine Lichtquelle zur Beleuchtung des mindestens einen Auges (112, 112') und eine Kamera (134) zur Erfassung der Augenbewegung aufweist.

15. Vorrichtung (110) nach einem der Ansprüche 13 oder 14, **dadurch gekennzeichnet, dass** die Vorrichtung (110) weiterhin mindestens eine der folgenden Einrichtungen umfasst:
- eine Refraktionsmesseinrichtung, die zur Erfassung der Refraktion des mindestens einen Auges (112, 112') eingerichtet ist;
- einen Bildschirm, der zur Darstellung des mindestens einen Zeichens in der ersten Entfernung (166) vor dem mindestens einen Auge (112, 112') eingerichtet ist; und/oder
- eine Projektionseinrichtung, die zur Abbildung des mindestens einen Zeichens auf das mindestens eine Auge (112, 112') des Nutzers eingerichtet ist.

16. Vorrichtung zur Datenverarbeitung, umfassend:
a) Mittel zum Darstellen mindestens eines Zeichens in mindestens einer ersten Entfernung (166) vor mindestens einem Auge (112, 112') eines Nutzers zur Stimulation der Akkommodation des mindestens einen Auges (112, 112');
b) Mittel zum Erfassen mindestens einer Augenbewegung des mindestens einen Auges (112, 112'); und
c) Mittel zum Ermitteln einer Refraktion des mindestens einen Auges (112, 112') bei der Akkommodation des mindestens einen Auges (112, 112') in die mindestens eine erste Entfernung (166);
d) gemeinsames Bestimmen der Akkommodation und der Vergenz des mindestens einen Auges (112, 112') durch
o Ermitteln einer Änderung der Refraktion des mindestens einen Auges (112, 112') bei der Akkommodation des mindestens einen Auges (112, 112') in die mindestens eine erste Entfernung (166) gegenüber der Akkommodation des mindestens einen Auges (112, 112') in mindestens eine zweite Entfernung (160); und
o Ermitteln der Vergenz des mindestens einen Auges (112, 112') aus der mindestens einen Augenbewegung des mindestens einen Auges (112, 112') bei der Akkommodation des mindestens einen Auges (112, 112') in die mindestens eine erste Entfernung (166),
**dadurch gekennzeichnet,**
**dass** das Ermitteln der Refraktion des mindestens einen Auges (112, 112') durch Erfassen der mindestens einen Augenbewegung des mindestens einen Auges (112, 112') in Abhängigkeit von dem mindestens einen Zeichen erfolgt, während ein Parameter des mindestens einen Zeichens verändert wird, wobei ein Zeitpunkt festgestellt wird, zu dem sich aus der mindestens einen Augenbewegung eine Erkennungsschwelle des Nutzers für das mindestens eine Zeichen ergibt, wobei die Refraktion des mindestens einen Auges (112, 112') aus dem zu dem Zeitpunkt festgelegten Parameter für das mindestens eine Zeichen ermittelt wird.

## Claims

1. Method (210) for jointly determining accommodation and vergence of at least one eye (112, 112') of a user, wherein the method (210) comprises the following steps:
a) presenting at least one symbol at at least one first distance (166) in front of at least one eye (112, 112') of a user for stimulating the accommodation of the at least one eye (112, 112');
b) registering at least one eye movement of the at least one eye (112, 112'); and
c) ascertaining a refraction of the at least one eye (112, 112') with the accommodation of the at least one eye (112, 112') at the at least one first distance (166);
d) jointly determining the accommodation and the vergence of the at least one eye (112, 112') by
o ascertaining a change in the refraction of the at least one eye (112, 112') with the accommodation of the at least one eye (112, 112') at the at least one first distance (166) in relation to the accommodation of the at least one eye (112, 112') at at least one second distance (160); and
o ascertaining the vergence of the at least one eye (112, 112') from the at least one eye movement of the at least one eye (112, 112') with the accommodation of the at least one eye (112, 112') at the at least one first distance (166);
**characterized**
**in that** the refraction of the at least one eye (112, 112') is ascertained by registering the at least one eye movement of the at least one eye (112, 112') on the basis of the at least one symbol while a parameter of the at least one symbol is altered, wherein a point in time is determined at which a recognition threshold of the user for the at least one symbol emerges from the at least one eye movement, wherein the refraction of the at least one eye (112, 112') is ascertained from the parameter for the at least one symbol defined at this point in time.

2. Method (210) according to Claim 1, **characterized in that** the joint determination of the accommodation and the vergence is implemented for both eyes (112, 112') of the user.

3. Method (210) according to either of Claims 1 and 2, **characterized in that** a newly ascertained value for the refraction of the at least one eye (112, 112') with the accommodation of the at least one eye (112, 112') at least at the second distance (160) is ascertained or **in that** a known value for the refraction of the at least one eye (112, 112') with the accommodation of the at least one eye (112, 112') at least at the second distance (160) is used.

4. Method (210) according to any of Claims 1 to 3, **characterized in that** the at least one first distance (166) is chosen for an accommodated state of the at least one eye (112, 112') and the at least second distance (160) is chosen for an unaccommodated state of the at least one eye (112, 112'), or **in that** the at least one first distance (166) is chosen for an unaccommodated state of the at least one eye (112, 112') and the at least second distance (160) is chosen for an accommodated state of the at least one eye (112, 112').

5. Method (210) according to any of Claims 1 to 4, **characterized in that** for the purposes of ascertaining the vergence of the at least one eye (112, 112'), an eye rotation (120, 120') of the at least one eye (112, 112') is registered while the at least one eye (112, 112') is accommodated at the at least one first distance (166).

6. Method (210) according to any of Claims 1 to 5, **characterized in that** the refraction of the at least one eye (112, 112') is ascertained by means of a refraction measuring device (144), which is configured to register the refraction of the at least one eye (112, 112').

7. Method (210) according to any of Claims 1 to 6, **characterized in that** the eye movement of the at least one eye (112, 112') has an eye movement metric, wherein the eye movement metric is selected from the group comprising a pursuit eye movement, an eye movement relating to microsaccades, and an optokinetic nystagmus.

8. Method (210) according to any of Claims 1 to 7, **characterized in that** the at least one symbol is presented by virtue of
- the at least one symbol being arranged at the first distance (166) in front of the at least one eye (112, 112');
- the at least one symbol being projected at the first distance (166) in front of the at least one eye (112, 112'); and/or
- the at least one symbol being projected onto the at least one eye (112, 112') such that the user can virtually recognize the at least one symbol at the first distance (166) in front of the at least one eye (112, 112').

9. Computer program comprising executable instructions for carrying out a method (210) for jointly determining accommodation and vergence of at least one eye (112, 112') of a user, wherein the method (210) comprises the following steps:
a) presenting at least one symbol at at least one first distance (166) in front of at least one eye (112, 112') of a user for stimulating the accommodation of the at least one eye (112, 112') by means of a device (124) configured to present the at least one symbol at at least the first distance (166) in front of at least the one eye (112, 112') of the user for stimulating the accommodation of the at least one eye (112, 112');
b) registering at least one eye movement of the at least one eye (112, 112') by means of an eye movement measuring device (132); and
c) ascertaining a refraction of the at least one eye (112, 112') with the accommodation of the at least one eye (112, 112') at the at least one first distance (166) by means of a refraction measuring device (144) and/or the eye movement measuring device (132);
d) jointly determining the accommodation and the vergence of the at least one eye (112, 112') by means of an evaluation unit (136) by way of
o ascertaining a change in the refraction of the at least one eye (112, 112') with the accommodation of the at least one eye (112, 112') at the at least one first distance (166) in relation to the accommodation of the at least one eye (112, 112') at at least one second distance (160); and
o ascertaining the vergence of the at least one eye (112, 112') from the at least one eye movement of the at least one eye (112, 112') with the accommodation of the at least one eye (112, 112') at the at least one first distance (166),
**characterized**
**in that** the refraction of the at least one eye (112, 112') is ascertained by registering the at least one eye movement of the at least one eye (112, 112') on the basis of the at least one symbol while a parameter of the at least one symbol is altered, wherein a point in time is determined at which a recognition threshold of the user for the at least one symbol emerges from the at least one eye movement, wherein the refraction of the at least one eye (112, 112') is ascertained from the parameter for the at least one symbol defined at this point in time.

10. Computer-readable data medium, on which the computer program according to Claim 9 is stored.

11. Data medium signal that transmits the computer program according to Claim 9.

12. Computer-readable medium comprising instructions that, upon execution by a computer, cause the latter to carry out the steps of a computer-implemented method for producing a spectacle lens for at least one eye (112, 112') of a user, wherein the spectacle lens is produced by processing a lens blank or a semifinished spectacle lens product, wherein the lens blank or the semifinished spectacle lens product is processed on the basis of refraction data (228), wherein the refraction data (228) take account of values for an accommodation and a vergence of at least one eye (112, 112') of the user, wherein a method (210) for jointly determining the accommodation and the vergence of the eye (112, 112') of the user is used, the method comprising the following steps:
a) presenting at least one symbol at at least one first distance (166) in front of at least one eye (112, 112') of a user for stimulating the accommodation of the at least one eye (112, 112');
b) registering at least one eye movement of the at least one eye (112, 112'); and
c) ascertaining a refraction of the at least one eye (112, 112') with the accommodation of the at least one eye (112, 112') at the at least one first distance (166);
d) jointly determining the accommodation and the vergence of the at least one eye (112, 112') by
o ascertaining a change in the refraction of the at least one eye (112, 112') with the accommodation of the at least one eye (112, 112') at the at least one first distance (166) in relation to the accommodation of the at least one eye (112, 112') at at least one second distance (160); and
o ascertaining the vergence of the at least one eye (112, 112') from the at least one eye movement of the at least one eye (112, 112') with the accommodation of the at least one eye (112, 112') at the at least one first distance (166),
**characterized**
**in that** the refraction of the at least one eye (112, 112') is ascertained by registering the at least one eye movement of the at least one eye (112, 112') on the basis of the at least one symbol while a parameter of the at least one symbol is altered, wherein a point in time is determined at which a recognition threshold of the user for the at least one symbol emerges from the at least one eye movement, wherein the refraction of the at least one eye (112, 112') is ascertained from the parameter for the at least one symbol defined at this point in time.

13. Apparatus (110) for jointly determining accommodation and vergence of at least one eye (112, 112') of a user, wherein the apparatus (110) comprises:
- a device (124) configured to present at least one symbol at at least a first distance (166) in front of at least one eye (112, 112') of a user for stimulating the accommodation of the at least one eye (112, 112');
- an eye movement measuring device (132) configured to register at least one eye movement of the at least one eye (112, 112'); and
- an evaluation unit (136) configured to jointly determine the accommodation from the change in refraction and the vergence from the eye movement by way of
o ascertaining a change in the refraction of the at least one eye (112, 112') with the accommodation of the at least one eye (112, 112') at the at least one first distance (166) in relation to the accommodation of the at least one eye (112, 112') at at least one second distance (160); and
o ascertaining the vergence of the at least one eye (112, 112') from the at least one eye movement of the at least one eye (112, 112') with the accommodation of the at least one eye (112, 112') at the at least one first distance (166),
**characterized**
**in that** the evaluation unit (136) is further configured to ascertain the refraction of the at least one eye (112, 112') by registering the at least one eye movement of the at least one eye (112, 112') on the basis of the at least one symbol while a parameter of the at least one symbol is altered, wherein a point in time is determined at which a recognition threshold of the user for the at least one symbol emerges from the at least one eye movement, wherein the refraction of the at least one eye (112, 112') is ascertained from the parameter for the at least one symbol defined at this point in time.

14. Apparatus (110) according to Claim 13, **characterized in that** the eye movement measuring device (132) comprises a light source for illuminating the at least one eye (112, 112') and a camera (134) for registering the eye movement.

15. Apparatus (110) according to either of Claims 13 and 14, **characterized in that** the apparatus (110) furthermore comprises at least one of the following devices:
- a refraction measuring device configured to register the refraction of the at least one eye (112, 112');
- a screen configured to present the at least one symbol at the first distance (166) in front of the at least one eye (112, 112'); and/or
- a projection device configured to image the at least one symbol on the at least one eye (112, 112') of the user.

16. Apparatus for data processing, comprising:
a) means for presenting at least one symbol at at least one first distance (166) in front of at least one eye (112, 112') of a user for stimulating the accommodation of the at least one eye (112, 112');
b) means for registering at least one eye movement of the at least one eye (112, 112'); and
c) means for ascertaining a refraction of the at least one eye (112, 112') with the accommodation of the at least one eye (112, 112') at the at least one first distance (166);
d) jointly determining the accommodation and the vergence of the at least one eye (112, 112') by
o ascertaining a change in the refraction of the at least one eye (112, 112') with the accommodation of the at least one eye (112, 112') at the at least one first distance (166) in relation to the accommodation of the at least one eye (112, 112') at at least one second distance (160); and
o ascertaining the vergence of the at least one eye (112, 112') from the at least one eye movement of the at least one eye (112, 112') with the accommodation of the at least one eye (112, 112') at the at least one first distance (166);
**characterized**
**in that** the refraction of the at least one eye (112, 112') is ascertained by registering the at least one eye movement of the at least one eye (112, 112') on the basis of the at least one symbol while a parameter of the at least one symbol is altered, wherein a point in time is determined at which a recognition threshold of the user for the at least one symbol emerges from the at least one eye movement, wherein the refraction of the at least one eye (112, 112') is ascertained from the parameter for the at least one symbol defined at this point in time.

## Revendications

1. Procédé (210) pour déterminer de manière commune l'accommodation et la vergence d'au moins un œil (112, 112') d'un utilisateur, dans lequel le procédé (210) comprend les étapes consistant à :
a) représenter au moins un signe à au moins une première distance (166) devant au moins un œil (112, 112') d'un utilisateur pour la stimulation de l'accommodation dudit au moins un œil (112, 112') ;
b) détecter au moins un mouvement oculaire dudit au moins un œil (112, 112') ; et
c) déterminer une réfraction dudit au moins un œil (112, 112') lors de l'accommodation dudit au moins un œil (112, 112') à ladite au moins une première distance (166) ;
d) déterminer de manière commune l'accommodation et la vergence dudit au moins un œil (112, 112') par
o détermination d'une modification de la réfraction dudit au moins un œil (112, 112') lors de l'accommodation dudit au moins un œil (112, 112') à ladite au moins une première distance (166) par rapport à l'accommodation dudit au moins un œil (112, 112') à au moins une deuxième distance (160) ; et
o détermination de la vergence dudit au moins un œil (112, 112') à partir dudit au moins un mouvement oculaire dudit au moins un œil (112, 112') lors de l'accommodation dudit au moins un œil (112, 112') à ladite au moins une première distance (166),
**caractérisé en ce que**
la détermination de la réfraction dudit au moins un œil (112, 112') est effectuée en détectant ledit au moins un mouvement oculaire dudit au moins un œil (112, 112') en fonction dudit au moins un signe, tout en modifiant un paramètre dudit au moins un signe, dans lequel on établit un instant où un seuil de reconnaissance de l'utilisateur pour ledit au moins un signe est obtenu à partir dudit au moins un mouvement oculaire, dans lequel la réfraction dudit au moins un œil (112, 112') est déterminée à partir du paramètre établi audit instant pour ledit au moins un signe.

2. Procédé (210) selon la revendication 1, **caractérisé en ce que** la détermination commune de l'accommodation et de la vergence est effectuée pour les deux yeux (112, 112') de l'utilisateur.

3. Procédé (210) selon l'une des revendications 1 ou 2, **caractérisé en ce qu'**une valeur nouvellement déterminée pour la réfraction dudit au moins un œil (112, 112') est déterminée lors de l'accommodation dudit au moins un œil (112, 112') à au moins la deuxième distance (160) ou **en ce qu'**une valeur connue est utilisée pour la réfraction dudit au moins un œil (112, 112') lors de l'accommodation dudit au moins un œil (112, 112') à au moins la deuxième distance (160).

4. Procédé (210) selon l'une des revendications 1 à 3, **caractérisé en ce que** ladite au moins une première distance (166) pour un état accommodé dudit au moins un œil (112, 112') et au moins la deuxième distance (160) est sélectionnée pour un état non accommodé dudit au moins un œil (112, 112'), ou **en ce que** ladite au moins une première distance (166) pour un état non accommodé dudit au moins un œil (112, 112') et au moins la deuxième distance (160) est sélectionnée pour un état accommodé dudit au moins un œil (112, 112').

5. Procédé (210) selon l'une des revendications 1 à 4, **caractérisé en ce que**, pour la détermination de la vergence dudit au moins un œil (112, 112'), une détection d'une rotation oculaire (120, 120') dudit au moins un œil (112, 112') est effectuée pendant l'accommodation dudit au moins un œil (112, 112') à ladite au moins une première distance (166).

6. Procédé (210) selon l'une des revendications 1 à 5, **caractérisé en ce que** la détermination de la réfraction dudit au moins un œil (112, 112') est effectuée au moyen d'un dispositif de mesure de réfraction (144) qui est conçu pour détecter la réfraction dudit au moins un œil (112, 112').

7. Procédé (210) selon l'une des revendications 1 à 6, **caractérisé en ce que** le mouvement oculaire dudit au moins un œil (112, 112') présente une métrique de mouvement oculaire, dans lequel la métrique de mouvement oculaire est choisie dans le groupe comprenant un mouvement de suivi oculaire ; un mouvement oculaire se rapportant à des microsaccades ; et un nystagmus optocinétique.

8. Procédé (210) selon l'une des revendications 1 à 7, **caractérisé en ce que** la représentation dudit au moins un signe est effectuée en faisant en sorte
- que ledit au moins un signe soit agencé à ladite première distance (166) devant ledit au moins un œil (112, 112') ;
- que ledit au moins un signe soit projeté à ladite première distance (166) devant ledit au moins un œil (112, 112') ; et/ou
- que ledit au moins un signe soit projeté sur ledit au moins un œil (112, 112') de manière à ce que l'utilisateur puisse reconnaître virtuellement ledit au moins un signe à ladite première distance (166) devant ledit au moins un œil (112, 112').

9. Programme informatique comprenant des instructions exécutables destinées à mettre en œuvre un procédé (210) pour déterminer de manière commune l'accommodation et la vergence d'au moins un œil (112, 112') d'un utilisateur, dans lequel le procédé (210) comprend les étapes consistant à :
a) représenter au moins un signe à au moins une première distance (166) devant au moins un œil (112, 112') d'un utilisateur pour la stimulation de l'accommodation dudit au moins un œil (112, 112') au moyen d'un dispositif (124) qui est conçu pour représenter ledit au moins un signe à au moins la première distance (166) devant au moins un œil (112, 112') de l'utilisateur pour la stimulation de l'accommodation dudit au moins un œil (112, 112') ;
b) détecter au moins un mouvement oculaire dudit au moins un œil (112, 112') au moyen d'un dispositif de mesure de mouvement oculaire (132) ; et
c) déterminer une réfraction dudit au moins un œil (112, 112') lors de l'accommodation dudit au moins un œil (112, 112') à ladite au moins une première distance (166) au moyen d'un dispositif de mesure de réfraction (144) et/ou du dispositif de mesure de mouvement oculaire (132) ;
d) déterminer de manière commune l'accommodation et la vergence dudit au moins un œil (112, 112') au moyen d'une unité d'évaluation (136) par
o détermination d'une modification de la réfraction dudit au moins un œil (112, 112') lors de l'accommodation dudit au moins un œil (112, 112') à ladite au moins une première distance (166) par rapport à l'accommodation dudit au moins un œil (112, 112') à au moins une deuxième distance (160) ; et
o détermination de la vergence dudit au moins un œil (112, 112') à partir dudit au moins un mouvement oculaire dudit au moins un œil (112, 112') lors de l'accommodation dudit au moins un œil (112, 112') à ladite au moins une première distance (166),
**caractérisé en ce que**
la détermination de la réfraction dudit au moins un œil (112, 112') est effectuée en détectant ledit au moins un mouvement oculaire dudit au moins un œil (112, 112') en fonction dudit au moins un signe, tout en modifiant un paramètre dudit au moins un signe, dans lequel on établit un instant où un seuil de reconnaissance de l'utilisateur pour ledit au moins un signe est obtenu à partir dudit au moins un mouvement oculaire, dans lequel la réfraction dudit au moins un œil (112, 112') est déterminée à partir du paramètre établi audit instant pour ledit au moins un signe.

10. Support de données lisible par ordinateur sur lequel est enregistré le programme d'ordinateur selon la revendication 9.

11. Signal porteur de données qui transmet le programme d'ordinateur selon la revendication 9.

12. Support lisible par ordinateur comprenant des instructions qui, lorsqu'elles sont exécutées par un ordinateur, amènent ce dernier à exécuter les étapes d'un procédé mis en œuvre par ordinateur pour fabriquer un verre de lunettes destiné à au moins un œil (112, 112') d'un utilisateur, dans lequel la fabrication du verre de lunettes est effectuée par usinage d'une ébauche de verre ou d'un produit semi-fini de verre de lunettes, dans lequel l'ébauche de lentille ou le produit semi-fini de verre de lunettes est usiné à l'aide de données de réfraction (228), dans lequel les données de réfraction (228) tiennent compte de valeurs concernant une accommodation et une vergence d'au moins un œil (112, 112') de l'utilisateur, dans lequel un procédé (210) est utilisé pour déterminer de manière commune l'accommodation et la vergence de l'œil (112, 112') de l'utilisateur, lequel procédé comprend les étapes consistant à :
a) représenter au moins un signe à au moins une première distance (166) devant au moins un œil (112, 112') d'un utilisateur pour la stimulation de l'accommodation dudit au moins un œil (112, 112') ;
b) détecter au moins un mouvement oculaire dudit au moins un œil (112, 112') ; et
c) déterminer une réfraction dudit au moins un œil (112, 112') lors de l'accommodation dudit au moins un œil (112, 112') à ladite au moins une première distance (166) ;
d) déterminer de manière commune l'accommodation et la vergence dudit au moins un œil (112, 112') par
o détermination d'une modification de la réfraction dudit au moins un œil (112, 112') lors de l'accommodation dudit au moins un œil (112, 112') à ladite au moins une première distance (166) par rapport à l'accommodation dudit au moins un œil (112, 112') à au moins une deuxième distance (160) ; et
o détermination de la vergence dudit au moins un œil (112, 112') à partir dudit au moins un mouvement oculaire dudit au moins un œil (112, 112') lors de l'accommodation dudit au moins un œil (112, 112') à ladite au moins une première distance (166),
**caractérisé en ce que**
la détermination de la réfraction dudit au moins un œil (112, 112') est effectuée en détectant ledit au moins un mouvement oculaire dudit au moins un œil (112, 112') en fonction dudit au moins un signe, tout en modifiant un paramètre dudit au moins un signe, dans lequel on établit un instant où un seuil de reconnaissance de l'utilisateur pour ledit au moins un signe est obtenu à partir dudit au moins un mouvement oculaire, dans lequel la réfraction dudit au moins un œil (112, 112') est déterminée à partir du paramètre établi audit instant pour ledit au moins un signe.

13. Dispositif (110) pour déterminer de manière commune l'accommodation et la vergence d'au moins un œil (112, 112') d'un utilisateur, dans lequel le dispositif (110) comprend :
- un dispositif (124) qui est conçu pour représenter au moins un signe à au moins une première distance (166) devant au moins un œil (112, 112') d'un utilisateur pour la stimulation de l'accommodation dudit au moins un œil (112, 112') ;
- un dispositif de mesure de mouvement oculaire (132) qui est conçu pour détecter au moins un mouvement oculaire dudit au moins un œil (112, 112') ; et
- une unité d'évaluation (136) qui est conçue pour déterminer de manière commune l'accommodation à partir de la modification de la réfraction et de la vergence résultant du mouvement oculaire, par
o détermination d'une modification de la réfraction dudit au moins un œil (112, 112') lors de l'accommodation dudit au moins un œil (112, 112') à ladite au moins une première distance (166) par rapport à l'accommodation dudit au moins un œil (112, 112') à au moins une deuxième distance (160) ; et
o détermination de la vergence dudit au moins un œil (112, 112') à partir dudit au moins un mouvement oculaire dudit au moins un œil (112, 112') lors de l'accommodation dudit au moins un œil (112, 112') à ladite au moins une première distance (166),
**caractérisé en ce que**
l'unité d'évaluation (136) est en outre conçue pour mettre en œuvre la détermination de la réfraction dudit au moins un œil (112, 112') en détectant ledit au moins un mouvement oculaire dudit au moins un œil (112, 112') en fonction dudit au moins un signe, tout en modifiant un paramètre dudit au moins un signe, dans lequel on établit un instant où un seuil de reconnaissance de l'utilisateur pour ledit au moins un signe est obtenu à partir dudit au moins un mouvement oculaire, dans lequel la réfraction dudit au moins un œil (112, 112') est déterminée à partir du paramètre établi audit instant pour ledit au moins un signe.

14. Dispositif (110) selon la revendication 13, **caractérisé en ce que** le dispositif de mesure de mouvement oculaire (132) comporte une source lumineuse destinée à éclairer ledit au moins un œil (112, 112') et une caméra (134) destinée à détecter le mouvement oculaire.

15. Dispositif (110) selon l'une des revendications 13 ou 14, **caractérisé en ce que** le dispositif (110) comprend en outre au moins l'un des dispositifs suivants :
- un dispositif de mesure de réfraction conçu pour détecter la réfraction dudit au moins un œil (112, 112') ;
- un écran conçu pour représenter ledit au moins un signe à la première distance (166) devant ledit au moins un œil (112, 112') ; et/ou
- un dispositif de projection conçu pour représenter ledit au moins un signe sur ledit au moins un œil (112, 112') de l'utilisateur.

16. Dispositif de traitement de données, comprenant :
a) des moyens destinés à représenter au moins un signe à au moins une première distance (166) devant au moins un œil (112, 112') d'un utilisateur pour la stimulation de l'accommodation dudit au moins un œil (112, 112') ;
b) des moyens destinés à détecter au moins un mouvement oculaire dudit au moins un œil (112, 112') ; et
c) des moyens destinés à déterminer une réfraction dudit au moins un œil (112, 112') lors de l'accommodation dudit au moins un œil (112, 112') à ladite au moins une première distance (166) ;
d) le fait de déterminer de manière commune de l'accommodation et de la vergence dudit au moins un œil (112, 112') par
o détermination d'une modification de la réfraction dudit au moins un œil (112, 112') lors de l'accommodation dudit au moins un œil (112, 112') à ladite au moins une première distance (166) par rapport à l'accommodation dudit au moins un œil (112, 112') à au moins une deuxième distance (160) ; et
o détermination de la vergence dudit au moins un œil (112, 112') à partir dudit au moins un mouvement oculaire dudit au moins un œil (112, 112') lors de l'accommodation dudit au moins un œil (112, 112') à ladite au moins une première distance (166),
**caractérisé en ce que**
la détermination de la réfraction dudit au moins un œil (112, 112') est effectuée en détectant ledit au moins un mouvement oculaire dudit au moins un œil (112, 112') en fonction dudit au moins un signe, tout en modifiant un paramètre dudit au moins un signe, dans lequel on établit un instant où un seuil de reconnaissance de l'utilisateur pour ledit au moins un signe est obtenu à partir dudit au moins un mouvement oculaire, dans lequel la réfraction dudit au moins un œil (112, 112') est déterminée à partir du paramètre établi audit instant pour ledit au moins un signe.
